# EUROPÄISCHE PATENTSCHRIFT

(11) **EP 1 454 133 B1**
(45) Veröffentlichungstag und Bekanntmachung des Hinweises auf die Patenterteilung: **16.04.2008**
(21) Anmeldenummer: 02792938.9
(22) Anmeldetag: 09.12.2002
(51) Int. Cl.: G01N 33/36, G01N 22/00, G01N 22/04

(54) **VERWENDUNG VON MIKROWELLEN IN DER SPINNEREIINDUSTRIE ZUR MESSUNG DER FASERBANDMASSE**
USE OF MICROWAVES IN THE SPINNING INDUSTRY
UTILISATION DE MICRO-ONDES DANS L'INDUSTRIE DE LA FILATURE

(30) Priorität: 11.12.2001 DE 10160757; 01.02.2002 DE 10204328
(43) Veröffentlichungstag der Anmeldung: 08.09.2004
(73) Patentinhaber: Rieter Ingolstadt Spinnereimaschinenbau AG, 85055 Ingolstadt (DE)
(72) Erfinder: DÄMMIG, Joachim, 85053 Ingolstadt (DE); CHERIF, Chokri, 85057 Ingolstadt (DE)
(74) Vertreter: Schlief, Thomas P.
(86) Internationale Anmeldenummer: PCT/EP2002/013907
(87) Internationale Veröffentlichungsnummer: WO 2003/050530

(56) Entgegenhaltungen:
- EP-A- 0 176 661
- WO-A-00/12974
- DE-A- 19 705 260
- PATENT ABSTRACTS OF JAPAN vol. 013, no. 001 (P-808), 6. Januar 1989 (1989-01-06) & JP 63 210757 A (NIPPON GLASS FIBER CO LTD), 1. September 1988 (1988-09-01)

## Beschreibung

Die Erfindung betrifft ein Verfahren zum Ermitteln der Bandmasse eines bewegten Faserverbandes an einer Spinnereivorbereitungsmaschine sowie eine entsprechende Spinnereivorbereitungsmaschine entsprechend den Oberbegriffen der unabhängigen Ansprüche 1 und 23.

In der Spinnereiindustrie wird beispielsweise aus Baumwolle in mehreren Prozeßschritten zuerst ein vergleichmäßigter Faserverband und schließlich als Endprodukt ein gedrehtes Garn produziert. Die der Garnherstellung vorgeordneten Spinnereivorbereitungsmaschinen, wie Karden und Strecken, haben insbesondere die Aufgabe, die Bandmasseschwankungen eines oder mehrerer Faserbänder - im folgenden zusammenfassend als Faserverband bezeichnet - auszuregulieren. Zu diesem Zweck sind beispielsweise an Strecken Bandsensoren angeordnet, welche die Bandmasse bzw. Bandmasseschwankungen messen und diese Informationen an eine Reguliereinheit weitergeben, die mindestens eines der Verzugsorgane des Streckwerks entsprechend ansteuert. Eine nach einem solchen Regulierprinzip arbeitende Strecke ist beispielsweise das Model RSB-D30 der Firma RIETER. Auch bei unregulierten Strecken sind Informationen hinsichtlich der Bandmasseschwankungen in vielen Fällen erwünscht. Ein entsprechender Sensor am Auslauf einer solchen Strecke gibt beispielsweise ein entsprechendes Abschaltsignal für die Maschine und/oder ein Warnsignal aus, wenn ein Schwellenwert der Bandmasse unter- bzw. überschritten wird.

Zur Messung der Bandmasse- bzw. Banddickenschwankungen sind insbesondere mechanische Abtastungen bekannt, die sich heutzutage in fast allen entsprechenden Maschinen durchgesetzt haben. Allerdings reicht die Dynamik dieser mechanischen Sensoren bei Liefergeschwindigkeiten von mehr als 1000 m/min nicht mehr aus. Zudem macht sich die notwendige starke mechanische Verdichtung vor dem mechanischen Sensor negativ auf die Verzugsfähigkeit bemerkbar.

Neben der mechanischen Abtastung der Banddickenschwankungen sind weitere Abtastprinzipien vorgeschlagen worden. So ist beispielsweise aus der US 2,942,303 sowie der DE 44 45 720 A1 bekannt, die Banddicke berührungslos mit durchdringender optischer Strahlung zu messen. Jedoch wird die Messgenauigkeit hierbei stark von den Umgebungseinflüssen, z.B. Temperatur, Feuchtigkeit und Schmutz, beeinflußt. Außerdem ist das Verfahren anfällig gegenüber Farbe sowie Reflektionseigenschaften des Faserverbandes.

Weitere bekannte berührungslose Messverfahren sind solche, die Ultraschallwellen verwenden. Ebenfalls bekannt sind kapazitiv oder pneumatisch arbeitende Messmethoden. Auch ist vorgeschlagen worden, Röntgenstrahlung oder γ-Strahlen zu verwenden. Allen diesen Verfahren ist jedoch gemeinsam, daß sie feuchteempfindlich sind. Es nützt daher wenig, daß klimatische Einflüsse wie die Temperatur und die relative Luftfeuchtigkeit sich in der Regel kompensieren lassen, um klimatische Einflüsse minimieren zu können. Das Problem der inhärenten Faserfeuchte läßt sich hierdurch nicht ohne weiteres beseitigen. Es sei hier nur erwähnt, daß Viskose beispielsweise bei 40 %-iger relativer Luftfeuchtigkeit eine Feuchte von ca. 13 % aufweist. Bei einer relativen Luftfeuchtigkeit von 90 % steigt dieser Wert auf 25 %. Zudem kann bei ein und derselben Baumwollpartie die Faserfeuchte bei gleichbleibenden Umgebungsbedingungen bis zu 5 % variieren. Auch nehmen die oberen Baumwollagen in einer Kanne, die einer Spinnereivorbereitungsmaschine zugestellt wird, mehr Feuchtigkeit auf als die darunter liegenden. Außerdem weisen die textilen Fasern durch die Veränderung der klimatischen Bedingungen innerhalb einer Spinnerei - z.B. morgens vs. mittags vs. abends - unterschiedliche Feuchte auf. Die genannten Einflüsse haben ihrerseits einen großen Einfluß auf das Messergebnis der Bandmasse und somit auf die Regulierungsgüte.

Die vorliegende Erfindung betrifft hingegen die Verwendung von Mikrowellen in der Spinnereiindustrie vor, um die Bandmasse eines im wesentlichen ungedrehten Faserverbandes zu messen bzw. eine Fremdstofferkennung durchzuführen.

In der WO 00/12974 A ist ein diesbezügliches Verfahren und eine entsprechende Vorrichtung beschrieben, wobei Fasermaterial in Form von Garn oder Faserbändern kontinuierlich durch einen Mikrowellenresonator geführt wird. Die Resonanzeigenschaften des Resonatorhohlraumes (Resonanzfrequenz, Güte) werden durch das Fasermaterial verändert. Aus der Resonanzfrequenzverschiebung und der Güteänderung (Dämpfung) lassen sich Feuchte- und Dichtewerte des Fasermaterials extrahieren. Auch die Temperatur des Fasermaterials kann gemessen werden, um eine Temperaturkompensation vornehmen zu können.

Es ist Aufgabe der Erfindung, die im wesentlichen berührungslose Bandmassenermittlung eines Faserverbandes bzw. generell Messungen an einem Faserverband mittels Mikrowellen zu verbessern.

Diese Aufgabe wird durch die Merkmale der unabhängigen Ansprüche 1 und 23 gelöst.

Wenn im Rahmen dieser Erfindung von der Messung der Bandmasse die Rede ist, fällt unter diese Formulierung ebenfalls die Messung äquivalenter Größen wie insbesondere des Substanzquerschnittes oder der Banddichte bzw. der Schwankungen dieser Größen.

Die Erfindung eignet sich insbesondere zur Messung an textilen, im wesentlichen ungedrehten, d.h. höchstens nur in äußerst geringem Maße gedrehten, Fasersträngen, wie beispielsweise Baumwollsträngen, die einer Spinnereivorbereitungsmaschine vorgelegt werden oder diese verlassen. Auch andere natürliche und synthetische Faserarten sind vermessbar, ebenso wie Mischungen aus natürlichen und synthetischen Fasern.

Erfindungsgemäß wird die Messfrequenz, mit der die in der Resonanzfrequenz angepaßten Mikrowellen-Resonanzsignale ausgekoppelt bzw. verarbeitet werden, auf die Einlaufgeschwindigkeit des in die Spinnereivorbereitungsmaschine einlaufenden Faserverbandes abgestimmt. Bei einer erfindungsgemäßen Alternative wird die Messfrequenz auf die Liefergeschwindigkeit des die Maschine verlassenden Faserverbandes abgestimmt. Mit anderen Worten wird eine maschinensynchrone Messfrequenz eingestellt.

Dies bedeutet, daß festgelegte Abtastlängen, z.B. 1,5 mm, unabhängig von der Bandgeschwindigkeit eingehalten werden. Diese längenorientierte Abtastung in vorgegebenen Abständen entlang des Faserverbandes (konstante Intervalllänge) verhindert insbesondere Schwebungen und vereinfacht die Auswertung der Messergebnisse. Zur Realisierung dieser variablen Mess- bzw. Abtastfrequenz kann ein externer Synchronisationseingang beim Detektor vorgesehen sein, der von der Bandgeschwindigkeit abhängige Synchronisationssignale erhält.

Eine alternative Methode ist eine zeitorientierte Abtastung, wobei die Messfrequenz von der Faserbandgeschwindigkeit abhängt. Diese Vorgehensweise entspricht der längenorientierten Abtastung, wobei nur ein anderes Bezugssystem gewählt ist.

Um eine eventuell zu grobe Auflösung des Messverfahrens auszugleichen, werden die Messungen entlang des Faserbandes bevorzugt in mehreren aufeinanderfolgenden, sich örtlich überlappenden Messabschnitten durchgeführt. Falls die Auflösung beispielsweise 1 cm beträgt, wird die Messfrequenz beispielsweise derart gewählt, daß alle 2 mm entlang des Faserbandes eine neue Messung durchgeführt wird. Somit wird 1 cm der Bandlänge in fünf Schritten abgetastet. Mit anderen Worten werden aufeinanderfolgende Messungen jeweils 2 mm in Bandrichtung gegeneinander versetzt vorgenommen, so daß jeder 2 mm lange Faserverbandabschnitt fünfmal erfaßt wird. Eine solche Messwertüberlappung reduziert die Integrationslänge und steigert die effektive Auflösung durch mathematische Berechnungen. Die reale Auflösung jeder Messung bleibt hierbei unverändert, im o.g. Beispiel also bei 1 cm.

Das genannte Verfahren zur Steigerung der effektiven Auflösung wird bevorzugt am Ausgang der Strecke eingesetzt. Bisher werden Spektrogramme von das Streckwerk verlassendem Faserband unterhalb von ca. 1 cm oder sogar von 10 cm interpoliert, da die Dynamik der eingesetzten mechanischen Abtastvorrichtungen zu gering ist. Daher ist ein real gemessener CV-Wert bei 1 cm und weniger von großem Interesse. Vorzugsweise wird daher, insbesondere auch am Eingang und/oder am Ausgang der Spinnereivorbereitungsmaschine, ein Spektrogramm des Faserverbandes mit Hilfe des mindestens einen Mikrowellensensors aufgenommen bzw. ein Teil eines solchen Spektrogramms - vorzugsweise zumindest im sehr kurzwelligen Bereich - ergänzt.

Der Einsatz von Mikrowellen entsprechend der Erfindung läßt sich beispielsweise dadurch realisieren, daß ein länglicher Faserverband mit einer Faserfeuchte in ein Probenvolumen eingebracht wird, das Teil mindestens eines Mikrowellenresonators ist. Mikrowellen werden von mindestens einem Mikrowellengenerator erzeugt und in den Resonator eingekoppelt, der entweder nach dem Reflexions- oder dem Transmissionsprinzip arbeitet. Aufgrund der Anwesenheit des feuchten Faserverbandes ändert sich die Resonanzfrequenz, d.h. die Eigenresonanz, im Vergleich zum leeren Resonator. Mittels eines Prozessors läßt sich die neue Resonanzfrequenz bestimmen und einstellen, wobei neben dieser Resonanzfrequenz auch die Dämpfung des Signals der ausgekoppelten Mikrowellen mit Hilfe eines entsprechend ausgebildeten Detektors gemessen wird. Anhand der Messsignale zur neuen Eigenresonanz läßt sich dann mit bekannten Verfahren die Faserfeuchte rechnerisch eliminieren.

Ein mögliches Verfahren zur Auswertung der Mikrowellensignale bei der Ermittlung der feuchteunabhängigen Dichte- bzw. Massemessung ist beispielsweise aus der EP 0 468 023 B1 bekannt, die sich jedoch im wesentlichen mit der Messung der dichteunabhängigen absoluten Feuchte von Stoffen beschäftigt. Das in der genannten Druckschrift beschriebene Verfahren läßt sich in seinen Grundzügen auf die feuchteunabhängige Ermittlung der Bandmasse bzw. Banddichte eines Faserverbandes übertragen. Der Offenbarungsgehalt der EP 0 468 023 B1 ist hiermit ausdrücklich miteingeschlossen.

Bei dem in der EP 0 468 023 B1 beschriebenen Verfahren wird eine im wesentlichen von der Bandmasse und der Faserfeuchte abhängige Messkurve erhalten, die Informationen hinsichtlich der verstimmten Resonanzfrequenz und der Halbwertsbreite enthält. Das Verfahren beruht im wesentlichen darauf, das von der Materialfeuchte abhängige Messsignal mit primären Messgrößen, wie der Halbwertsbreite und der Resonanzfrequenz, in Verbindung zu bringen. Das Messsignal wird dann mit einer für das Fasermaterial spezifischen, abgespeicherten Kalibrationskurve verglichen. Unter Berücksichtigung der Halbwertsbreite und der Resonanzfrequenz des Mikrowellensignals bei leerem Resonator. (ohne Messgut) läßt sich dann entweder die dichteunabhängige Feuchte oder die feuchteunabhängige Dichte des Messguts ermitteln, wobei für die vorliegende Erfindung insbesondere letzteres von Interesse ist.

Ein weiteres, etwas aufwendigeres Verfahren ist in den DE 197 05 260 A1 und DE 197 34 978 A1 insbesondere für Zigarettenstränge beschrieben, in denen die Einstrahlung von Mikrowellen zweier Frequenzen beschrieben ist. Auch der Offenbarungsgehalt dieser beiden Druckschriften ist hiermit explizit eingeschlossen (s. hierzu auch Ansprüche 13 und 36).

Mikrowellen lassen sich gemäß einer bevorzugten Ausführungsform der Erfindung zur Ermittlung der Bandmasse eines Faserverbandes unter Berücksichtigung der Faserfeuchte anwenden. Hierbei wird vorteilhafterweise die Bandmasse feuchteunabhängig ermittelt, bevorzugtermaßen indem die Faserfeuchte bei der Ermittlung der Bandmasse rechnerisch eliminiert wird. Alternativ kann auch von einer konstanten Grundfaserfeuchte ausgegangen werden, wobei beispielsweise die zu vermessende Faserart und festgelegte Umgebungsbedingungen als Referenz angenommen werden. Gleichfalls sind weitere Parameter für die Annahme einer gewissen Grundfaserfeuchte wählbar. So sind beispielsweise die Decklagen des zu verarbeitenden Fasermaterials als Referenz heranzuziehen. Von der Grundfaserfeuchte ausgehend können dann die Abweichungen der Bandmasse ermittelt werden. Diese Methode entspricht demnach einer relativen Berücksichtigung der Faserfeuchte des Faserverbandes.

Besonders bevorzugt wird das Mikrowellenmessprinzip bei Regulierstrecken verwendet. Die ermittelten Werte für die Bandmasse bzw. die Bandmasseschwankungen werden einer Reguliereinheit zugeführt, die entsprechend ein Verzugsorgan einer Spinnereivorbereitungsmaschine ansteuert, welche einen kontinuierlich durchlaufenden Faserverband vergleichmäßigt. Beispielsweise wird der Faserverband in einem Streckwerk einer Strecke in einem Vorverzugsfeld und einem anschließenden Hauptverzugsfeld verstreckt. Die Verzugsfelder werden hierbei durch jeweils zwei Walzenpaare gebildet, wobei der Faserverband zwischen den Walzen eines solchen Klemmwalzenpaares geklemmt wird. In Verstreckungsrichtung nehmen hierbei die Umfangsgeschwindigkeiten der Walzen der einzelnen Walzenpaare zu. Durch Ansteuerung beispielsweise des Eingangs- und des mittleren von drei aufeinanderfolgenden Walzenpaaren wird deren Umfangsgeschwindigkeit verändert und somit auch der Verzug. Auf diese Weise sind Bandmasseschwankungen bzw. Banddickenschwankungen ausregulierbar.

Die Erfindung läßt sich ebenfalls mit Vorteil bei Karden einsetzen. Hier ist beispielsweise die Ersetzung des üblicherweise verwendeten Wegmesssensors bei den Tastwalzen (eine der beiden Tast- bzw. Abzugswalzen ist radial auslenkbar, wobei die Auslenkung ein Maß für die Banddicke ist) durch den mindestens einen Mikrowellensensor vorteilhaft. Die Verzugsorgane der Karde können dann entsprechend reguliert werden. Alternativ oder zusätzlich ist eine Regelung der Kardenspeisung möglich. Die Erfindung ist auch bei regulierten Karden, die am Ausgang ein reguliertes Streckwerk für den dann als Faservlies bezeichneten Faserverband aufweisen, oder bei Strecken einsetzbar. Gleichfalls läßt sich die Erfindung bei Kämmmaschinen mit einem am Ausgang angeordneten regulierten Streckwerk einsetzen, wobei ggf. diesem regulierten Streckwerk ein unreguliertes Streckwerk vorgeschaltet sein kann. Bei Karden und Kämmmaschinen ist das Streckwerk vorteilhafterweise in Form eines Moduls dem Ausgang dieser Spinnereivorbereitungsmaschinen zustellbar. Die Bezeichnung "Modul" bedeutet, daß die Karde bzw. Kämmmaschine einerseits und das Streckwerk andererseits keine gesamte, einheitliche Maschine bilden, sondern einen Maschinenverbund. Eine entsprechende Anpassung aufeinander ist notwendig.

Prinzipiell läßt sich die Erfindung insbesondere am Ein- und/oder Ausgang eines Streckwerks einer Spinnereivorbereitungsmaschine verwenden. Wird der Mikrowellensensor am Einlauf der Spinnereivorbereitungsmaschine eingesetzt und die Messwerte bzw. die Ergebnisse der Messwertauswertung der Regulierung zugeführt, spricht man von einer Einlaufregulierung. Bei einem Einsatz des Mikrowellensensors am Auslauf der Maschine handelt es sich um eine Auslaufregulierung. Mit dieser lassen sich insbesondere langwellige, wiederkehrende Bandmasseschwankungen ausregulieren. Auch ist eine Kombination von Einlauf- und Auslaufregulierung vorteilhaft, die eine sog. vermaschte Regelung bildet. Ein Beispiel ist in der EP 0 176 661 A2 beschrieben, deren Offenbarungsgehalt hiermit explizit einbezogen ist.

Es ist ebenfalls vorteilhaft, mittels eines Mikrowellensensors am Ausgang der Spinnereivorbereitungsmaschine eine Plausibilitätskontrolle für einen mechanischen Sensor am Ausgang durchzuführen. Mit anderen Worten wird der mechanische Sensor mit Hilfe des Mikrowellensensors überprüft, so daß beispielsweise bei Erreichen eines vorgewählten A%-Grenzwertes (langwellige Bandschwankungen) eine automatische Korrektur der Maschineneinstellwerte oder eine Laborüberprüfung des verzogenen Faserverbandes vorgenommen wird, um anschließend manuell die Maschine - beispielsweise die Klemmlinienabstände von Streckwerkswalzen - entsprechend einzustellen.

Alternativ oder zusätzlich zu einer Ausregulierung der Bandmasseschwankungen ist die Erfindung auch zur Überwachung des ein- und/oder auslaufenden Faserverbandes einsetzbar. Insbesondere bei unregulierten Strecken kann dann beispielsweise die Güte der mechanischen Einstellungen in Abhängigkeit von verschiedenen Fasermaterialien mit Hilfe des Mikrowellensensors festgestellt werden. Gleichfalls ist ein Abstellen der Maschine und/oder die Ausgabe eines Warnsignals bei Unter- oder Überschreiten eines Schwellenwertes der Bandmasse des auslaufenden Faserverbandes realisierbar.

Die Mikrowellenmesstechnik läßt sich gleichfalls als Überwachungsorgän zur Detektion eines Bandbruchs einsetzen. Eine solche Überwachung kann z.B. am Einlauf einer Strecke erfolgen, beispielsweise durch Messung des Faserverbandes über eine bestimmte Anzahl von Perioden, wobei bei Unterschreiten eines vorgegebenen Schwellenwertes die Maschine abschaltet. Auch am Auslauf der Strecke oder bei anderen Spinnereivorbereitungsmaschinen ist eine derartige Überwachung möglich. Vorteilhafterweise übernimmt der mindestens eine Mikrowellensensor sowohl die Bandmasseerfassung als auch die Erfassung eines Bandbruchs, da auch im letzteren Fall ein Schwellenwert hinsichtlich der Bandmasse unterschritten wird.

Mit Vorteil wird neben der Fasermasse auch die Faserfeuchtigkeit und/oder die Fasertemperatur erfaßt, die beispielsweise mittels IR-Strahlen an den entsprechenden Abschnitten gemessen wird. Auch diese Größen können dann zur Maschinensteuerung verwendet werden. Eine hohe Faserfeuchte, die insbesondere häufig bei Decklagen von aus Spinnkannen abgezogenen Faserbändern auftreten kann, beeinflußt das Verzugsverhalten in relativ starkem Maße. Auch kann Wickelbildung eine Folge zu hoher Faserfeuchte sein. Es ist daher vorteilhaft, auch die Faserfeuchte mit in die Steuerung/Regelung der Verzugsorgane einzubeziehen. Ähnliches gilt für die Fasertemperatur.

Besonders bevorzugt wird der Faserverband im wesentlichen schwingungsfrei durch den mindestens einen Resonator geführt. Auf diese Weise lassen sich Messwertverfälschungen vermeiden. Eine vorteilhafte Möglichkeit zur Realisierung einer solchen Schwingungsfreiheit bzw. -reduzierung wird durch eine seitliche Führung, d.h. quer zum Faserverband realisiert. Der Faserverband wird entweder nur von einer Seite oder von mehreren Seiten geführt (trotz einer solchen Führung handelt es um ein berührungsloses Messprinzip).

Vorzugsweise durchläuft der Faserverband den mindestens einen Resonator unter Anspannung in Längsrichtung - vorteilhafterweise mit einer geringen Anspannkraft, so daß der Faserverband hierdurch nicht verzogen wird. Eine solche Anspannung wird bevorzugt durch Führungsmittel vor und hinter dem mindestens einen Resonator realisiert. Die Führungsmittel können hierbei bevorzugt auch eine Ausrichtung der Faserbänder in Faserbandquerrichtung übernehmen.

Führungsmittel zur Führung und/oder Anspannung können vorteilhafterweise durch je ein den Faserverband klemmendes Transportwalzenpaar vor und hinter dem mindestens einen Resonator realisiert werden, wobei die Walzen zur Verbesserung der Klemmwirkung vorzugsweise onduliert sind.

Alternativ oder zusätzlich ist dem mindestens einen Resonator mindestens ein in Umfangsrichtung offener oder geschlossener Trichter vorgeschaltet, durch den der Faserverband oder ein Gruppe von Faserbändern - wobei mehrere Gruppen den Faserverband bilden - verdichtet und in Querrichtung geführt werden. Statt eines Trichters sind beispielsweise auch Leitbleche, Leitstangen o. dgl. verwendbar.

Alternativ oder zusätzlich wird der Faserverband oder einzelne, den Faserverband bildenden Gruppen von Faserbändern vor dem mindestens einen Resonator über ein als Verdichtungselement ausgebildetes Führungsmittel mit quer zur Faserverbandlängsrichtung ansteigenden Führungsflächen geführt. Somit rutschen die Faserbänder zur Mitte hin und werden verdichtet. Ein solches Führungsmittel ist vorzugsweise im wesentlichen als Doppelkegel mit ineinander zugewandten und ineinander übergehenden Spitzen ausgebildet und stellt somit eine Führungsrolle dar. Zudem kann ein derartiges Führungsmittel drehbar ausgebildet sein, um einen Transport des Faserverbandes in Faserlängsrichtung zu erleichtern.

Bevorzugt ist mindestens ein in Faserverbandquerrichtung verlaufender Druckstab als alternatives oder zusätzliches Führungsmittel vor dem mindestens einen Resonator angeordnet. Vorteilhafterweise ist ein solcher Druckstab der zuvor beschriebenen Führungsrolle nachgeordnet, bevor der Faserverband in den Resonator eintritt.

In weiteren Ausgestaltungen kann die gewünschte Anspannung auch unter Mitwirkung der Streckwerkswalzen realisiert werden. Wenn der Sensor beispielsweise unmittelbar vor dem Streckwerk angeordnet ist, kann das dem Sensor nachgeschaltete Eingangswalzenpaar des Streckwerks im Zusammenspiel mit beispielsweise einem Transportwalzenpaar vor dem Sensor die Anspannung bewirken. Eine Anordnung des Sensors mit kurzem Abstand vor dem Streckwerk bietet sich im übrigen bevorzugt an, da das zu verstreckende Textilmaterial - im Gegensatz zum Stand der Technik bei Abtastung mit beispielsweise mechanischen Tastwalzen - zuvor nicht komprimiert und daher auch nicht über eine längere Wegstrecke bis zum Streckwerk wieder ausgebreitet werden muß.

Wenn hingegen der Sensor dem Streckwerk nachgeschaltet ist, kann einerseits das ihm vorgeschaltete Lieferwalzenpaar des Streckwerks und andererseits ein nachgeschaltetes Kalanderwalzenpaar für die Anspannung des verstreckten Faserverbandes sorgen.

Bevorzugt wird der Faserverband in mindestens zwei Gruppen durch einen oder mehrere Resonatoren geführt, die in Querrichtung einen Abstand aufweisen. Hierzu können die Faserbänder entweder schon am Einlauf der Spinnereivorbereitungsmaschine - vorzugsweise beim Abziehen aus der Maschine vorgelegten Spinnkannen - in mindestens zwei räumlich voneinander getrennten Gruppen einlaufen, oder der Faserverband wird vor Durchlaufen des mindestens einen Resonators in die mindestens zwei Gruppen getrennt. Durch eine derartige Teilung des Faserverbandes kann ggf. die Messgenauigkeit des Mikrowellensensors gesteigert werden, da nur ein Teil des Faserverbandes jeweils ein Probenvolumen des mindestens einen Resonators durchläuft. Außerdem wird die genannte Führung der Faserbänder erleichtert.

Die genannten mindestens zwei Gruppen von Faserbändern werden vorzugsweise nach Durchlaufen des mindestens einen Resonators aufeinander zu geführt, um anschließend in ein Streckwerk der Spinnereivorbereitungsmaschine einzulaufen, in welchem sie bevorzugt im wesentlichen parallel zueinander verstreckt werden.

Um eine Relativbewegung zwischen Faserbändern in dem mindestens Resonator zu verhindern, die eine Weitergabe von verfälschten Informationen an die Reguliereinheit und damit zu Fehlverzügen führen würde, werden die Faserbänder bevorzugt entsprechend geführt. Hierzu sind vorteilhafterweise Umlenkelemente vorgesehen, die derart positioniert sind, daß die von entsprechenden Abschnitten verschiedener Faserbänder durchlaufenen Wegstrecken vom Resonator zum Streckwerk im wesentlichen gleich lang sind. Zu diesem Zweck sind vorteilhafterweise für jedes Faserband mindestens zwei Umlenkelemente vorgesehen, die in Faserverbandlängs- und - querrichtung gegeneinander versetzt angeordnet sind. Auf diese Weise läßt sich die Länge jeder Wegstrecke für die einzelnen Faserbänder auf das gleiche Maß einstellen.

Die Umlenkelemente sind weiterhin bevorzugt drehbar ausgebildet, um die Reibung zwischen Faserbändern und Umlenkelementen zu reduzieren.

In einer bevorzugten Ausführungsform werden die Faserbänder nebeneinander und parallel laufend durch den mindestens einen Mikrowellensensor geführt. Die Faserbänder können hierbei einen im wesentlichen konstanten Abstand zueinander aufweisen oder sich auch berühren. Eine Aufteilung in zwei oder mehrere Gruppen von Faserbändern entfällt hierbei. Hierdurch wird u.a. ein konstruktiv einfacher Mikrowellensensor realisiert. Eine solche Führung der Faserbänder hat den Vorteil, daß diese sich vorteilhafterweise im Resonatorraum homogen verteilen. Zur Realisierung einer solchen homogenen Verteilung der Faserbänder im Resonator bzw. im Resonatorraum können die zugeführten Faserbänder je nach räumlicher Anordnung bei der Zuführung verdichtet und/oder ausgebreitet werden.

In einer weiteren Ausgestaltung der Erfindung werden die Faserbänder nicht nur im Resonator, sondern vom Einlauf bis zum Auslauf nebeneinander und in Draufsicht im wesentlichen parallel laufend durch die Spinnereivorbereitungsmaschine geführt. Hierdurch werden insbesondere Reibungsverluste der Faserbänder durch Umlenkung und Verdichtung verhindert.

Es ist von Vorteil, wenn das Material zumindest des Probenvolumenabschnitts, der mit dem Faserverband in Berührung kommt, im wesentlichen abriebfest ausgebildet ist, um genaue Messergebnisse über einen langen Zeitraum zu erhalten und eine Langlebigkeit des Sensors zu gewährleisten.

Da sich immer wieder Fasern aus dem Faserverband lösen können (Faserflug), ist es zweckmäßig, das vom Faserverband durchlaufene Probenvolumen in Zeitabständen zu reinigen. Insbesondere bietet es sich an, hierzu Preßluft oder Unterdruck einzusetzen, die auf das Probenvolumen einwirken. Alternativ oder zusätzlich ist zumindest das Probenvolumen des mindestens einen Resonators während eines Faserverbandstillstandes verschiebbar, beispielsweise in Faserverbandquerrichtung verfahrbar, ausgebildet, um eine leichtere Zugänglichkeit für eine automatische oder manuelle Reinigung des Probenvolumens in einer Reinigungsposition des mindestens einen Resonators zu realisieren.

Vorteilhafte Weiterbildungen sind durch die Merkmale der Unteransprüche gekennzeichnet.

Im folgenden werden Ausführungsbeispiele der Erfindung anhand der Figuren erläutert. Es zeigen:
- Figur 1: eine erste Ausführungsform einer Strecke zum Verstrecken eines Faserverbandes;
- Figur 2: eine schematische Seitenansicht einer zweiten Ausführungsform einer Strecke;
- Figur 3: die Strecke der Figur 2 in Draufsicht;
- Figur 4a: einen Ausschnitt einer dritten Ausführungsform einer Strecke in Draufsicht mit sechs vorgelegten Faserbändern;
- Figur 4b: eine Sicht entlang einem Schnitt 1-1 gemäß der Figur 4a, und
- Figur 5: eine schematische Darstellung einer Kombination einer Karde oder Kämmmaschine mit einem Streckwerk.

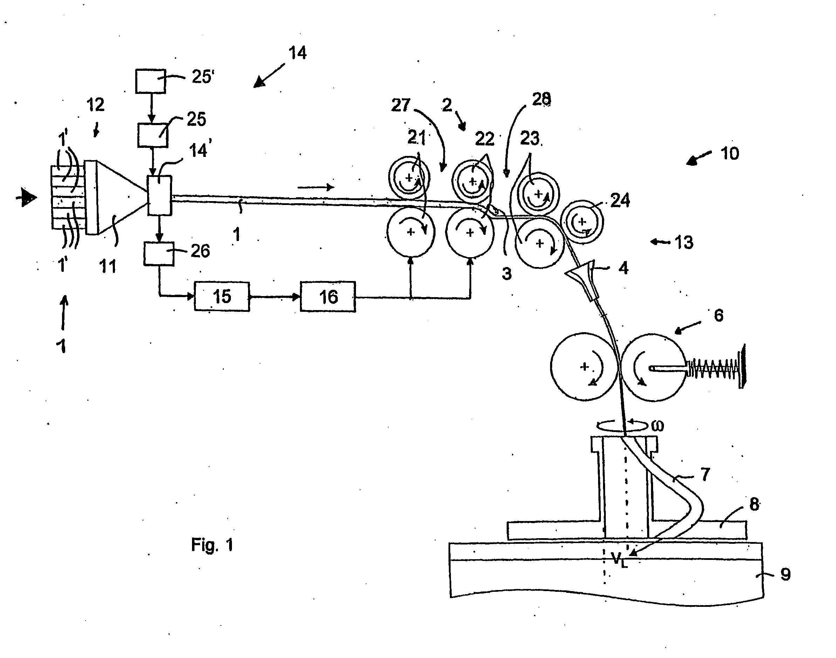

Die grundsätzliche Funktionsweise einer Strecke 10 - als Beispiel einer Spinnereivorbereitungsmaschine - wird nachfolgend anhand der Figur 1 erläutert. Gemäß diesem Beispiel werden mehrere, im wesentlichen ungedrehte Faserbänder 1 der Strecke 10 nebeneinander vorgelegt. Es ist ebenfalls möglich, der Strecke 10 nur ein Faserband 1' zuzuführen. In beiden Fällen wird für das vorgelegte Fasermaterial im Rahmen dieser Erfindung der Terminus Faserverband verwendet, der im folgenden das Bezugszeichen 1 trägt. Am Eingang der Strecke 10 ist ein Trichter 11 angeordnet, der die Faserbänder 1' bzw. den Faserverband 1 verdichtet. Alternativ können andere Verdichtungseinrichtungen verwendet werden. Ebenso ist denkbar, daß auf eine Verdichtung gänzlich verzichtet wird. Nach Durchlaufen einer weiter unten beschriebenen Abtastvorrichtung bzw. einem Sensor wird der Faserverband 1 in ein Streckwerk 2 geführt, welches das Kernstück der Strecke 10 bildet. Übliche Streckwerke weisen in der Regel ein Vorverzugsfeld und ein Hauptverzugsfeld auf. Bei unregulierten Strecken ist während des Verzugsvorgangs sowohl der Vorverzug als auch der Hauptverzug konstant. Bei regulierten Strecken erfolgt hingegen eine Ausregulierung durch Veränderung der Verzugshöhe. In einem regulierten Streckwerk ließe sich dazu sowohl der Vor- als auch der Hauptverzug verändern, gewählt wird aber fast immer der Hauptverzug. Der Grund liegt darin, daß der Hauptverzug größer ist als der Vorverzug, so daß eine genauere Regulierung vorgenommen werden kann.

Das Streckwerk 2 weist in der Regel drei Verzugsorgane bzw. Walzenpaare auf, zwischen denen der eigentliche Verzug stattfindet. Diese sind das Eingangswalzenpaar 21, das mittlere Walzenpaar 22 und das Ausgangs- oder auch Lieferwalzenpaar 23, die sich mit in dieser Reihenfolge jeweils gesteigerter Umfangsgeschwindigkeit drehen. Durch diese unterschiedlichen Umfangsgeschwindigkeiten der Walzenpaare wird der Faserverband 1 entsprechend dem Verhältnis der Umfangsgeschwindigkeiten verzogen. Das Eingangswalzenpaar 21 und das mittlere Walzenpaar 22 bilden hierbei das genannte Vorverzugsfeld 27, das mittlere Walzenpaar 22 und das Lieferwalzenpaar 23 das genannte Hauptverzugsfeld 28. Üblicherweise wird zusätzlich ein Druckstab 3 im Hauptverzugsfeld angebracht, der den aus den Faserbändern bestehenden Faserverband 1 umlenkt und somit für eine bessere Führung der Fasern sorgt, insbesondere der nicht zwischen zwei Walzenpaaren geklemmten Fasern (sog. schwimmende Fasern). Der verzogene Faserverband 1 wird mit Hilfe einer Umlenkoberwalze 24 und eines Vliestrichters 4 zusammengefaßt und über ein Kalanderwalzenpaar 6 und einen geschwungenen Bandkanal 7, der in einem sich mit der Winkelgeschwindigkeit ω drehenden Drehteller 8 angeordnet ist, mit einer Geschwindigkeit V_{L} in einer Kanne 9 abgelegt.

Zum Ausgleich der Bandmasseschwankungen an regulierten Strecken durchlaufen die vorgelegten Faserbänder üblicherweise einen dem Streckwerk 2 vorgelagerten Sensor 14, welcher die Banddicke laufend registriert und in Form von elektrischen Spannungssignalen zuerst an einen Speicher 15, der den Weg- bzw. den Zeitunterschied zwischen dem Passieren des Sensors 14 und dem Eintritt in das Streckwerk 2 berücksichtigt (FIFO-Speicher = First-In-First-Out-Speicher), und dann nach Ablauf dieser Zeitdifferenz an eine Auswerte- und Reguliereinheit 16 weiterleitet. Das Messsignal wird demnach im Speicher 15 zwischengespeichert, damit die Auswerte- und Reguliereinheit 16 nach einer vorgegebenen Zeit bzw. einem vom Faserverband 1 definiert zurückgelegten Weg die Regulierung einschaltet, welche die Masseschwankungen durch Veränderung der Umfangsgeschwindigkeiten des mittleren Walzenpaares 22 und ggf. des Eingangswalzenpaares 21 ausgleicht (s. Pfeilrichtungen). Dieser Einsatzpunkt wird als Regeleinsatzpunkt bezeichnet. Der Ausgleich der Masseschwankungen im Hauptverzugsfeld 28 wird durch die Veränderung der Drehzahl eines Regelmotors (nicht dargestellt) erreicht, der die Eingangswalzen 21 und die Mittelwalzen 22 antreibt, wobei die Drehzahl des Motors für den Antrieb der Lieferwalzen 23 konstant gehalten wird.

Gemäß der vorliegenden Erfindung arbeitet die Abtastvorrichtung bzw. der Sensor 14 mit Mikrowellen, wobei bevorzugt das Resonatorprinzip Anwendung findet. Zu diesem Zweck umfaßt der Sensor 14 mindestens ein Mikrowellengenerator 25, dessen Frequenz mittels eines Prozessors 25' variabel einstellbar ist, und der Mikrowellen in einen Hohlraum bzw. ein Probenvolumen mindestens eines Resonators 14' einkoppelt, der ebenfalls Teil des Mikrowellensensors ist. Während der Abtastung durchläuft der Faserverband 1 das Probenvolumen. Die durch das Probenvolumen hindurchtransportierten Abschnitte des Faserverbandes 1 beeinflussen hierbei entsprechend ihrer jeweiligen Dicke bzw. Masse sowie Feuchte die Resonatorfrequenz und die Dämpfung, die sich in einer Änderung der Amplitude und der Halbwertsbreite des aus dem Resonator 14' ausgekoppelten und mittels eines entsprechend ausgebildeten, ebenfalls einen Teil des Sensors 14 bildenden Mikrowellendetektors 26 detektierten Signals niederschlägt. Aus den detektierten Signalen läßt sich dann die Bandmasse des abgetasteten Faserverbandabschnittes berechnen, wobei die Feuchte dieses Abschnitts herausgerechnet werden kann, damit anschließend die Ausregulierung der Bandmasseschwankungen mit Hilfe der Auswerte- und Reguliereinheit 16 vorgenommen werden kann. Die Erfindung betrifft neben der Verwendung von Mikrowellen auch die entsprechenden Verfahren sowie entsprechende Vorrichtungen, die auf diese Weise textile, im wesentlichen ungedrehte Faserverbände messen.

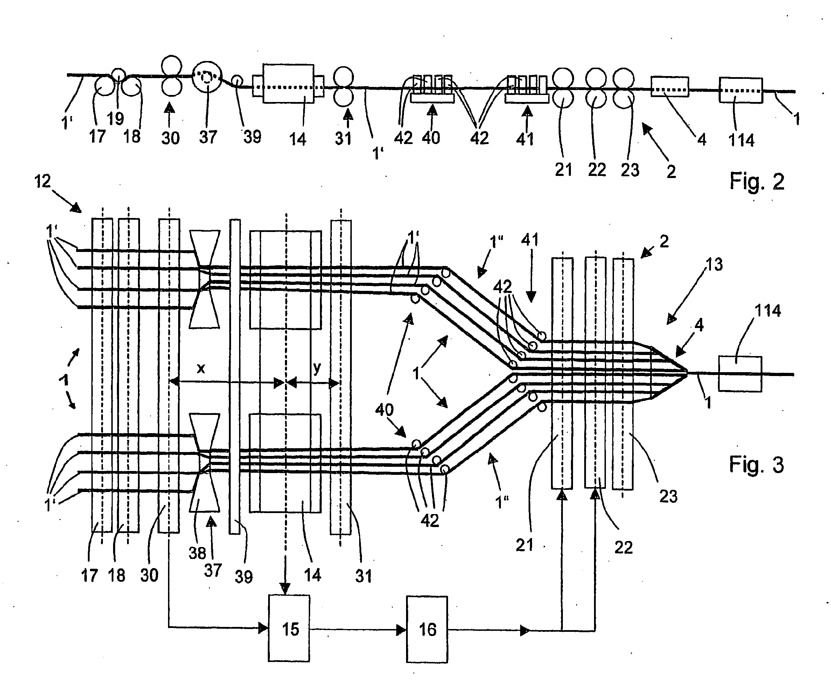

In Fig. 2 in Seitenansicht und in Fig. 3 in Draufsicht ist ein Ausführungsbeispiel einer erfindungsgemäßen Spinnereivorbereitungsmaschine in Gestalt einer Strecke 10 dargestellt. Der Faserverband 1 wird in zwei Gruppen 1 ", die jeweils vier im wesentlichen parallel laufende Faserbänder 1' umfassen, zum Einlauf 12 der Strecke 10 transportiert, üblicherweise durch Abziehen aus der Maschine 10 vorgelegten Spinnkannen (nicht dargestellt). In Transportrichtung werden die Faserbänder 1' über zwei hintereinander angeordnete metallische Zuführwalzen 17, 18 geführt, auf die mittig mehrere, parallel zu den Walzenachsen nebeneinander angeordnete metallische Belastungswalzen 19 aufgelegt sind (in Fig. 3 der Übersichtlichkeit halber weggelassen). Falls ein Faserband 1' reißen sollte, wird ein elektrischer Kontakt über die Walzen 17, 18, 19 geschlossen und der Faserband-Transport gestoppt.

In der Fig. 3 sind die Faserbänder 1' der Übersichtlichkeit halber als durchgehende Linien dargestellt, obwohl sie in der Draufsicht teilweise verdeckt sein würden, so zum Beispiel im Sensor 14 oder im Streckwerk 2.

An die Walzen 17, 18, 19 schließt sich ein als Führungsmittel ausgebildetes Transportwalzenpaar 30 an, welches von zwei in Faserverband- bzw. Transportquerrichtung beabstandeten, als Verdichtungselementen 37 ausgebildeten weiteren Führungselementen gefolgt ist. Über jedes Verdichtungselement 37 wird jeweils eine Gruppe 1" von vier Faserbändern 1' geführt. Die Verdichtungselemente 37 sind als Doppelkegel ausgebildet, die mit ihrer Spitze einander zugewandt sind und ineinander übergehen. An den seitlich ansteigenden Führungsflächen 38 rutschen die Faserbänder 1' abwärts und werden somit verdichtet.

Alternativ zu den Verdichtungselementen 37 kann beispielsweise auch jeweils ein Trichter (nicht dargestellt) verwendet werden, der ebenfalls zur Verdichtung von jeweils vier Faserbändern 1' dient.

Auf die Verdichtungselemente 37 folgt ein Druckstab 39, unter den die Faserbänder 1' hergeführt werden, um nachfolgend in das Probenvolumen eines Resonators eines Mikrowellensensors 14 einzutreten. Dieser Sensor 14 weist vorliegend zwei Probenvolumina auf, wobei beispielsweise von beiden Seiten der Strecke 10 jeweils eine Faserband-Gruppe 1" in ein entsprechendes Probenvolumen des Sensors 14 einführbar ist. Andere Ausbildungen des Sensors 14 sind selbstverständlich möglich.

Dem Sensor 14 nachgeschaltet ist ein weiteres Transportwalzenpaar 31, das - im Zusammenspiel mit dem Transportwalzenpaar 30 (sowie dem Verdichtungselement 37 und dem Druckstab 39) - ebenfalls zur Führung und insbesondere zum Erzeugen einer Anspannung des Faserverbandes 1 dient. Schwingungen der Faserbänder 1' werden insbesondere dadurch vermieden, daß der Abstand x des Transportwalzenpaares 30 bzw. der Abstand y des Transportwalzenpaares 31 zum Sensor 14 gering gewählt ist.

Stromabwärts des Transportwalzenpaares 31 sind Umlenkelemente 42 für die Faserbänder 1' vorgesehen, welche die Faserbänder 1 wieder zusammenführen. Insbesondere ist es Zweck dieser Umlenkelemente 42, daß die einzelnen Faserbänder 1' vom Sensor 14 bis zum Streckwerk 2 Wegstrecken gleicher Länge zurücklegen. Auf diese Weise wird gewährleistet, daß die zu gleichen Zeiten hinsichtlich der Bandmasse vermessenen Faserbandabschnitten auch zu gleichen Zeiten das Streckwerk 2 durchlaufen und somit ein exakter Verzug erreicht werden kann. Würden sich die Faserbänder 1' auf ihrem Weg vom Sensor 14 zum Streckwerk 2 relativ zueinander bewegen, wären Fehlverzüge die Folge.

In der in den Fig. 2 und 3 dargestellten Ausführungsform sind jeweils vier Umlenkelemente 42 zu einer Vierergruppe 40, 41 zusammengefaßt, wobei ein Umlenkelement 42 einer Gruppe 40, 41 für die Umlenkung eines Faserbandes 1' vorgesehen ist. Die Umlenkelemente 42 jeder Vierergruppe 40, 41 sind fluchtend und zur Faserverband-Transportrichtung in einem Winkel von ca. 45° verlaufend angeordnet. Für jedes Faserband 1' sind insgesamt zwei Umlenkelemente 42 vorgesehen, die in Faserverband-Transportrichtung sowohl längs- als auch querversetzt angeordnet sind, so daß ein Faserband 1' zweimal auf dem Weg vom Sensor 14 zum Streckwerk 2 umgelenkt wird. Die Umlenkelemente 42 der Gruppen 40, 41 sind hierbei derart angeordnet, daß das außenlaufende Faserband 1' einer Faserband-Gruppe 1" in der stromaufwärts angeordneten Vierergruppe 40 zu einem späteren Zeitpunkt und in der stromabwärts angeordneten Vierergruppe 41 zu einem früheren Zeitpunkt umgelenkt wird als das innenlaufende Faserband 1'. In der Summe sind die durchlaufenen Wegstrecken jedes Faserbandes 1' vom Sensor 14 zum Streckwerk 2 gleich lang.

In der Ausführungsform der Fig. 2 und 3 sind die Umlenkelemente 42 als vertikal ausgerichtete Rundstäbe ausgebildet. Die Umlenkmittel 42 können um eine vertikale Achse drehbar ausgebildet sein, um die Reibung für die Faserbänder 1' zu reduzieren.

Nach Umlenkung durch die Umlenkelemente 42 laufen die Faserbänder 1' in das Streckwerk 2 ein und werden verzogen. Hierbei werden - wie zuvor beschrieben - die Messsignale des Sensors 14 (der Mikrowellen-Detektor ist nicht dargestellt) über den Zwischenspeicher 15 zur Auswerte- und Reguliereinheit 16 übermittelt, welche eine Steuerung des Eingangs- und Mittelwalzenpaares 21, 22 veranlaßt (Einlaufregulierung). Dem Streckwerk 2 nachgeordnet ist der verdichtende Trichter 4 und anschließend ein weiterer Mikrowellensensor 114, der beispielsweise zur Bandkontrolle am Auslauf 13 des Streckwerks 2 dient. Dieser Sensor 114 kann insbesondere derart ausgebildet sein, daß er eine Abschaltung der Maschine 10 und/oder die Ausgabe eines Warnsignals veranlaßt, wenn der verzogene Faserverband 1 nicht die gewünschte Qualität aufweist. Ebenfalls ist eine Auslaufregulierung (nicht dargestellt) mit Hilfe des am Auslauf 13 angeordneten Sensors 114 möglich.

In den Figuren 2 und 3 ist jeweils nur ein Sensor 14, 114 (der Übersichtlichkeit halber jeweils ohne Mikrowellengeneratoren und -detektoren) vor bzw. nach dem Streckwerk 2 dargestellt. Ebenfalls ist es möglich, mehrere Sensoren 14 bzw. 114 einzusetzen, die nur einen Teil des Faserverbandes 1 hinsichtlich der Bandmasse vermessen. So ist es beispielsweise möglich, daß anstelle des in den Fig. 2 und 3 dargestellten Sensors 14 mit seinen zwei Probenvolumina zwei Sensoren 14 verwendet werden, wobei ein solcher Sensor jeweils eine Faserband-Gruppe 1" mißt.

Gleichfalls ist es nicht zwingend, daß die Faserbänder 1' in einer Ebene - wie insbesondere aus der Fig. 3 ersichtlich - geführt werden. Der Transportquerschnitt der Faserbänder 1' kann auch anders gewählt werden. Beispielsweise können jeweils zwei Faserbänder 1' nebeneinander und übereinander oder gegeneinander versetzt durch das Probenvolumen geführt werden.

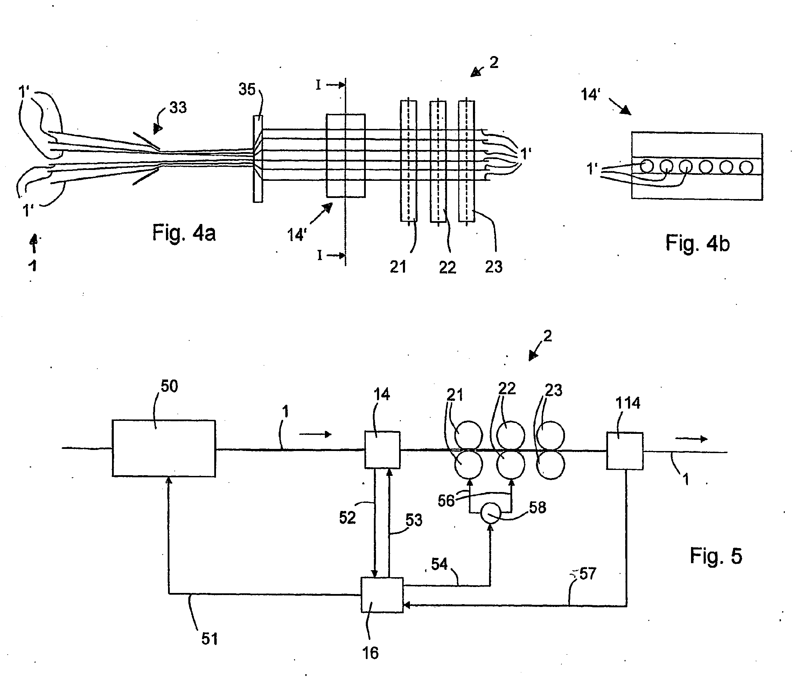

In den Fig. 4a (Draufsicht) und in einem kleinen Ausschnitt in Fig. 4b (Sicht in Richtung 1-1 der Figur 4a) ist eine alternative Ausführungsform einer Strecke schematisch dargestellt. Sechs vorgelegte Faserbänder 1' werden in einem Trichter oder zwei in Faserbandtransportrichtung aufeinander zulaufenden Leitblechen 33 - alternativ zwei Leitstangen - zusammengeführt. Mittels eines nachfolgenden Ausbreitungselements 35 werden die Faserbänder 1' derart ausgebreitet, daß jeweils zwei nebeneinander laufende Faserbänder 1' im wesentlichen gleich beabstandet angeordnet sind. Das Ausbreitungselement 35 ist beispielsweise als nach oben gebogener Rundstab ausgebildet. In alternativen Varianten werden die Faserbänder 1' auf im wesentlich konstanten Querabstand zusammengeführt, wie beispielsweise mit Hilfe der Verdichtungselemente 38 gemäß der Fig. 2 und 3. Wesentlich ist hier, daß die Faserbänder 1' mit homogener Verteilung den Resonatorraum durchlaufen. In der Figur 4b ist dies anschaulich dargestellt: Die sechs Faserbänder 1' haben im wesentlichen einen konstanten Abstand quer zu ihrer Transportrichtung. Die Faserbänder 1' können sich auch berühren. Bei gleicher Transportgeschwindigkeit treten hierbei keine störenden Reibungseffekte auf.

Wie der Fig. 4a weiterhin zu entnehmen ist, ist die von den Faserbändern 1' durchlaufene Wegstrecke im Anschluß an den Sensor 14 bis zum Streckwerk 2 kurz. Bei herkömmlichen Strecken hingegen, die mit mechanischer Bandabtastung und daher mit einer Bandkomprimierung arbeiten, ist diese Wegstrecke wesentlich länger ausgebildet, da sich z.B. Viskose nach einer Komprimierung relativ schlecht öffnet. Die Folge ist, daß hierfür eine verhältnismäßig große Distanz zwischen mechanischer Abtastvorrichtung und Streckwerk vorgesehen sein muß. Bei der berührungslosen Abtastung mittels Mikrowellen ist diese Zwischenschaltung einer längeren Distanz nicht notwendig, so daß der Sensor 14 unmittelbar vor dem Streckwerk 2 angeordnet sein kann. Die gesamte Maschinenlänge läßt sich somit ggf. deutlich reduzieren.

Dem Streckwerk 2 der Fig. 4a ist vorzugsweise ein Vliestrichter, ein Bandtrichter und anschließend ein Kalanderwalzenpaar nachgeschaltet (nicht dargestellt), wobei ein weiterer Mikrowellensensor zwischen diesen verschiedenen Bauteilen angeordnet sein kann.

In Fig. 5 ist eine Kombination einer Karde 50 und einem nachgeschalteten regulierten Streckwerk 2 dargestellt, zwischen denen ein Mikrowellensensor 14 angeordnet ist. Die Messwerte des Sensors 14 werden über eine Signalleitung 52 einer Auswerte- und Reguliereinheit 16 (ein Speicher 15 wie in den Fig. 1 und 3 ist fortgelassen) zugeführt und dort ausgewertet. Die Auswerte- und Reguliereinheit 16 kann dann über die Signalleitungen 51 bzw. 54 eine Regulierung der Karde 50 (Regulierung der Kardenverzugsorgane und/oder der Kardenspeisung) bzw. eine Steuerung der als Walzen ausgebildeten Verzugsorgane 21, 22 des Streckwerks 2 initiieren. Bei der Steuerung des Streckwerks 2 wird über die Signalleitung 54 ein Regelmotor 58 mit einem nachgeschalteten Differentialgetriebe (nicht dargestellt) zur Ansteuerung des Antriebs der Verzugsorgane 21, 22 über Signalleitungen 56 verwendet. Der Antrieb für die Verzugsorgane 23 (wie auch in Fig. 1) ist nicht dargestellt.

Dem Streckwerk 2 ist ein zweiter Mikrowellensensor 114 nachgeschaltet, der nicht nur eine Bandauslaufkontrolle durchführen kann, sondern auch zur Kalibrierung des Sensors 14 herangezogen werden kann. Hierzu ist eine Signalleitung 53 von der Auswerte- und Reguliereinheit 16 zum Sensor 14 vorgesehen. Die beiden Sensoren 14, 114 lassen sich auf diese Weise aufeinander anpassen.

Der Sensor 114 kann ebenfalls dazu eingesetzt werden, die Verzugsorgane des Streckwerks 2 zu regeln, um insbesondere langwellige Bandschwankungen auszugleichen. Zusammen mit der Steuerung aufgrund der Messsignale vom Sensor 14 wird hierdurch eine sog. vermaschte Regelung realisiert.

Statt der Karde 50 kann eine Kämmmaschine an ihrer Stelle vorgesehen sein, der ebenfalls ein reguliertes Streckwerk nachgeschaltet ist. Zwischen der Kämmmaschine und dem regulierten Streckwerk kann zudem ein unreguliertes Streckwerk (nicht dargestellt) vorgesehen sein. Die Kämmmaschine kann mittels der Auswerte- und Reguliereinheit 16 - wie die Karde - geregelt werden, während das regulierte Streckwerk 2 gesteuert und/oder geregelt wird.

Die Erfindung erstreckt sich ebenfalls auf die Verwendung, ein entsprechendes Verfahren sowie eine entsprechende Vorrichtung zur Erkennung von Fremdstoffen in textilem Faserverband, wobei dieser sowohl im wesentlichen ungedreht (Faservlies, Faserband) oder gedreht (als Garn) vorliegen kann. Das Prinzip zur Auswertung der Mikrowellensignale ist hierbei ähnlich dem zuvor für den im wesentlichen ungedrehten Faserverband vorgestellten. Fremdstoffe können - in Abhängigkeit von der Dielektrizitätskonstante des Fremdstoffes - beispielsweise zu einem höheren Bandmassewert als dem tatsächlichen führen. Das gemessene Signal kann hierbei charakteristisch für den jeweiligen Fremdstoff sein, wobei beispielsweise bei der rechnergestützten Signalauswertung auf abgespeicherte charakteristische Werte (z.B. in Form einer Wissensbasis) zurückgegriffen werden kann. Die Fremdstoffe können hierbei metallischer oder vegetabiler Art sein. Die Fremdstoffen lassen sich insbesondere dann gut feststellen, wenn ihre Dielektrizitätskonstante deutlich von derjenigen des Fasermaterials abweicht. Die Faserfeuchte kann wie zuvor beschrieben berücksichtigt werden.

## Patentansprüche

1. Verfahren zum Ermitteln der Bandmasse eines bewegten Faserverbandes an einer Spinnereivorbereitungsmaschine (10) mit mehreren aufeinander folgenden Verzugsorganen (21, 22, 23) zum Verstrecken des Faserverbandes (1), wobei das Verfahren folgende Schritte umfasst:
- es werden Mikrowellen in mindestens einen Resonator (14') eines Mikrowellensensors (14; 114) eingekoppelt,
- es wird der Faserverband (1) derart durch oder entlang des mindestens einen Resonators (14') geführt, dass sich die Eigenresonanz aufgrund der sich ändernden Bandmasse und Feuchte des Faserverbandes (1) verändert,
- es wird die Mikrowellenfrequenz durch prozessorgesteuerte Abstimmung an die veränderte Eigenresonanz angepasst,
- es werden die aus dem mindestens einen Resonator (14') ausgekoppelten, in der Frequenz angepassten Mikrowellen hinsichtlich Frequenzverstimmung und Dämpfung detektiert,
- es wird aus der Frequenzverstimmung und der Dämpfung die Bandmasse unter rechnerischer Berücksichtigung der Feuchte des Faserverbandes (1) mit Hilfe eines Prozessors berechnet,
**dadurch gekennzeichnet, dass** die Abtastfrequenz, mit welcher der durch den Resonator geführte Faserverband abgetastet wird, derart auf die Einlaufgeschwindigkeit des in die Spinnereivorbereitungsmaschine (10) einlaufenden oder die Liefergeschwindigkeit des die Spinnereivorbereitungsmaschine (10) verlassenden Faserverbandes (1) abgestimmt wird, dass eine von der Bandgeschwindigkeit unabhängige Abtastlänge ein gehalten wird.

2. Verfahren nach Anspruch 1, **dadurch gekennzeichnet, dass** die Abtastfrequenz einer festgelegten, vorzugsweise konstanten, Abtastlänge angepasst wird

3. Verfahren nach Anspruch 1, **dadurch gekennzeichnet, dass** die Abtastfrequenz einer festgelegten Zeitspanne angepasst wird die von der Faserverbandgeschwindigkeit abhängt.

4. Verfahren nach einem der vorhergehenden Ansprüche, **dadurch gekennzeichnet, dass** die pro Messung jeweils einen bestimmten Faserverbandabschnitt erfassende Abtastung in mehreren gegeneinander verschobenen und sich überlappenden Messungen entlang des Faserverbandes (1) durchgeführt wird.

5. Verfahren nach einem der vorhergehenden Ansprüche, **dadurch gekennzeichnet, dass** ein Spektrogramm oder ein Teil eines Spektrogramms des Faserverbandes (1') anhand der mittels des mindestens einen Mikrowellensensors (14, 114) erhaltenen Messwerte erstellt bzw. ergänzt wird.

6. Verfahren nach einem der vorhergehenden Ansprüche, **dadurch gekennzeichnet, dass** die der jeweiligen Bandmasse entsprechende Resonanzfrequenz sowie die Halbwertsbreite des Signals bei dieser Resonanzfrequenz ausgewertet wird.

7. Verfahren nach einem der vorhergehenden Ansprüche, **dadurch gekennzeichnet, dass** die Mikrowellen-Resonanzmessungen am Einlauf (12) und/oder Auslauf (13) eines Streckwerks (2) der Spinnereivorbereitungsmaschine (10) vorgenommen werden.

8. Verfahren nach einem der vorhergehenden Ansprüche, **dadurch gekennzeichnet, dass** die Bandmasseschwankungen am Einlauf (12) und/oder am Auslauf (13) überwacht und ggf. die Spinnereivorbereitungsmaschine (10) bei Unter- oder Überschreiten von Schwellenwerten der Bandmasse bzw. Bandmasseschwankungen abgeschaltet und/oder ein Warnsignal ausgegeben wird.

9. Verfahren nach einem der vorhergehenden Ansprüche, **dadurch gekennzeichnet, dass** der Mikrowellensensor (14, 114) zur Erfassung von Bandbrüchen des Faserverbandes (1) oder eines Faserbandes (1') des Faserverbandes (1) ausgebildet ist.

10. Verfahren nach einem der vorhergehenden Ansprüche, **dadurch gekennzeichnet, dass** anhand der berechneten Werte für die Bandmasse eine Reguliereinheit (16) der Spinnereivorbereitungsmaschine (10) im Rahmen einer Einlaufregulierung zumindest eines der Verzugsorgane (21, 22, 23) zur Ausregulierung der Bandmasseschwankungen steuert.

11. Verfahren nach einem der vorhergehenden Ansprüche, **dadurch gekennzeichnet, dass** anhand der berechneten Werte für die Bandmasse eine Reguliereinheit (16) der Spinnereivorbereitungsmaschine (10) im Rahmen einer Auslaufregulierung zumindest eines der Verzugsorgane (21, 22, 23) zur Ausregulierung der Bandmasseschwankungen regelt.

12. Verfahren nach Anspruch 10 und Anspruch 11, **dadurch gekennzeichnet, dass** die Einlauf- und Auslaufregulierung eine vermaschte Regelung (gleichzeitige Steuerung und Regelung) bilden.

13. Verfahren nach einem der vorhergehenden Ansprüche, **dadurch gekennzeichnet, dass** die lokale Fasertemperatur des Faserverbandes (1) ermittelt wird und dass die lokale Fasertemperatur und/oder die mit Hilfe des mindestens einen Mikrowellensensors ermittelte Faserfeuchte zusätzlich zur ermittelten Faserdichte zur Steuerung der Spinnereivorbereitungsmaschine herangezogen wird.

14. Verfahren nach Anspruch 13, **dadurch gekennzeichnet, dass** ein Spektrogramm des Faserverbandes (1') am Einlauf und/oder am Auslauf der Spinnereivorbereitungsmaschine aufgenommen wird.

15. Verfahren nach einem der vorhergehenden Ansprüche, **dadurch gekennzeichnet, dass** der Faserverband (1) im wesentlichen schwingungsfrei durch den mindestens einen Resonator (14') geführt wird.

16. Verfahren nach einem der vorhergehenden Ansprüche, **dadurch gekennzeichnet, dass** der Faserverband (1) seitlich geführt durch den mindestens einen Resonator (14') transportiert wird.

17. Verfahren nach einem der vorhergehenden Ansprüche, **dadurch gekennzeichnet, dass** der Faserverband (1) durch den mindestens einen Resonator (14') mit einer Anspannung geführt wird, wobei die Anspannung durch Führungsmittel (30, 31; 37, 39) vor und hinter dem mindestens einen Resonator (14') realisiert wird.

18. Verfahren nach einem der vorhergehenden Ansprüche, **dadurch gekennzeichnet, dass** mehrere Faserbänder (1') vom Einlauf (12) bis zum Auslauf (13) nebeneinander und in Draufsicht im wesentlichen parallel laufend durch die Spinnereivorbereitungsmaschine geführt werden.

19. Verfahren nach einem der vorhergehenden Ansprüche, **dadurch gekennzeichnet, dass** der Faserverband (1) oder einzelne, den Faserverband (1) bildende Gruppen (1") von Faserbändern (1') vor Durchlaufen des mindestens einen Resonators (14') durch mindestens einen Trichter oder durch Leitelemente, z.B. Leitblechen oder Leitstangen, hindurch geführt werden.

20. Verfahren nach einem der vorhergehenden Ansprüche, **dadurch gekennzeichnet, dass** der Faserverband (1) oder einzelne, den Faserverband (1) bildende Gruppen (1") von Faserbändern (1') vor Durchlaufen des mindestens einen Resonators (14') über mindestens ein Führungsmittel (37) mit zu den Seiten ansteigenden Führungsflächen (38) geführt werden, wobei der Faserverband (1) oder die Faserbänder (1') mindestens einer Gruppe (1") verdichtet werden.

21. Verfahren nach einem der vorhergehenden Verfahrensansprüche, **dadurch gekennzeichnet, dass** der mindestens eine Resonator (14') in Zeitabständen mit Pressluft oder einem Saugstrom gereinigt wird.

22. Verfahren nach einem der vorhergehenden Verfahrensansprüche, **dadurch gekennzeichnet, dass** zumindest der den Faserverband (1) aufnehmende Abschnitt des mindestens einen Resonators (14') während eines Faserverbandstillstandes für eine automatische oder manuelle Reinigung aus seiner Messposition in eine Reinigungsposition verschoben wird.

23. Spinnereivorbereitungsmaschine, insbesondere Karde (50), Strecke (10) oder Kämmmaschine, insbesondere zur Durchführung des Verfahrens nach einem der vorhergehenden Ansprüche, mit mindestens einem Sensor (14; 114) zur Messung der Bandmasse von kontinuierlich bewegtem Faserverband (1), wobei der mindestens eine Sensor (14; 114) umfasst:
- mindestens einen Mikrowellengenerator (25) zur Erzeugung von Mikrowellen prozessorgesteuerter, variabler Frequenzen,
- mindestens einen Mikrowellenresonator (14'), in den die Mikrowellen veränderbarer. Frequenz einkoppelbar und auskoppelbar sind,
- ein Probenvolumen zur Aufnahme des Faserverbandes (1) als Teil des Mikrowellenresonators (14'),
- einen Prozessor zur Abstimmung der eingekoppelten Mikrowellenfrequenz auf die durch die Einbringung des Faserverbandes (1) in das Probenvolumen veränderte Eigenresonanz des Mikrowellenresonators (14'),
- eine Auswerteeinheit zur Auswertung der ausgekoppelten Mikrowellen-Resonanzsignale hinsichtlich der Bandmasse des Faserverbandes (1) unter rechnerischer Berücksichtigung der Faserverbandfeuchte, **dadurch gekennzeichnet, dass** der Sensor darauf eingerichtet ist, die Abtastfrequenz, mit welcher der durch den Resonator geführte Faserverband abgetastet wird, auf die Einlaufgeschwindigkeit des in die Spinnereivorbereitungsmaschine (10) einlaufenden oder die Liefergeschwindigkeit des die Spinnereivorbereitungsmaschine (10) verlassenden Faserverbandes (1) so abzustimmen, dass eine von besagter Geschwindigkeit unabhängige Abtastlänge eingehalten wird, wobei ein externer Synchronisationseingang vorgesehen ist, der von der Einlauf- oder der Liefergeschwindigkeit abhängige Synchronisationssignale erhält.

24. Spinnereivorbereitungsmaschine nach Anspruch 23, **dadurch gekennzeichnet, dass** die Abtastfrequenz einer festgelegten, vorzugsweise konstanten, Abtastlänge angepasst ist.

25. Spinnereivorbereitungsmaschine nach Anspruch 23, **dadurch gekennzeichnet**, die Abtastfrequenz einer festgelegten Zeitspanne angepasst ist die von der Faserverbandgeschwindigkeit abhängt.

26. Spinnereivorbereitungsmaschine nach einem der vorhergehenden Vorrichtungsansprüche, **dadurch gekennzeichnet, dass** sie derart ausgelegt ist, dass die pro Messung jeweils einen bestimmten Faserverbandabschnitt erfassende Abtastung in mehreren gegeneinander verschobenen und sich überlappenden Messungen entlang des Faserverbandes (1) durchgeführt wird.

27. Spinnereivorbereitungsmaschine nach einem der vorhergehenden Vorrichtungsansprüche, **dadurch gekennzeichnet, dass** der mindestens eine Sensor (14) am Einlauf der Spinnereivorbereitungsmaschine angeordnet ist.

28. Spinnereivorbereitungsmaschine nach einem der vorhergehenden Vorrichtungsansprüche, **dadurch gekennzeichnet, dass** der mindestens eine Sensor (114) am Auslauf der Spinnereivorbereitungsmaschine angeordnet ist.

29. Spinnereivorbereitungsmaschine nach einem der vorhergehenden Vorrichtungsansprüche, **dadurch gekennzeichnet, dass** der mindestens eine Sensor (14) mit einer Reguliereinheit (16) verbunden ist, die anhand der Messwerte zur Bandmasse des Faserverbandes (1) mindestens ein Verzugsorgan (21, 22, 23) der Spinnereivorbereitungsmaschine steuert und/oder regelt.

30. Spinnereivorbereitungsmaschine nach einem der vorhergehenden Vorrichtungsansprüche, **dadurch gekennzeichnet, dass** mehrere Faserbänder nebeneinander und parallel laufend durch den mindestens einen Mikrowellensensor (14) führbar sind.

31. Spinnereivorbereitungsmaschine nach einem der vorhergehenden Vorrichtungsansprüche, **dadurch gekennzeichnet, dass** mehrere Faserbänder vom Einlauf bis zum Auslauf nebeneinander und in Draufsicht im wesentlichen parallel laufend durch die Spinnereivorbereitungsmaschine führbar sind.

32. Spinnereivorbereitungsmaschine nach einem der vorhergehenden Vorrichtungsansprüche, **dadurch gekennzeichnet, dass** Führungsmittel (30, 31; 37, 39; 21; 23) vor bzw. hinter dem Sensor (14; 114) zur Führung des Faserverbandes (1) mit einer Anspannung vorgesehen sind.

33. Spinnereivorbereitungsmaschine nach Anspruch 32, **dadurch gekennzeichnet, dass** die Führungsmittel (30, 31; 37, 39; 21; 23) rotierende Walzenpaare (30, 31; 21; 23) umfassen, zwischen denen der Faserverband (1) klemmbar ist.

34. Spinnereivorbereitungsmaschine nach Anspruch 32, **dadurch gekennzeichnet, dass** der Abstand (x, y) des dem Sensor (14) vor- und/oder nachgeordneten Walzenpaares (30, 31) zum Sensor (14) gering ist.

35. Spinnereivorbereitungsmaschine nach einem der vorhergehenden Vörrichtungsansprüche, **dadurch gekennzeichnet, dass** die Führungsmittel (30, 31; 37, 39; 21; 23) mindestens ein als Trichter, als Leitbleche oder als Leitstangen ausgebildetes Verdichtungselement vor dem mindestens einen Resonator (14') zum Zusammenführen des Faserverbandes (1) oder einzelner Gruppen (1") von Faserbändern (1') des Faserverbandes (1) umfassen.

36. Spinnereivorbereitungsmaschine nach einem der vorhergehenden Vorrichtungsansprüche, **dadurch gekennzeichnet, dass** die Führungsmittel (30, 31; 37, 39; 21; 23) mindestens ein Verdichtungselement (37) mit quer zur Faserverbandlängsrichtung ansteigenden Führungsflächen (38) zum Zusammenführen des Faserverbandes (1) oder einzelner Gruppen (1") von Faserbändern (1') des Faserverbandes (1) umfassen.

37. Spinnereivorbereitungsmaschine nach Anspruch 34, **dadurch gekennzeichnet, dass** das mindestens eine Verdichtungselement (37) im wesentlichen als Doppelkegel mit ineinander zugewandten und ineinander übergehenden Spitzen ausgebildet ist.

38. Spinnereivorbereitungsmaschine nach einem der Ansprüche 32 bis 37, **dadurch gekennzeichnet, dass** die Führungsmittel (30, 31; 37, 39; 21; 23) einen Druckstab (39) vor dem Sensor (14) umfassen.

39. Spinnereivorbereitungsmaschine nach einem der Ansprüche 32 bis 38, **dadurch gekennzeichnet, dass** zumindest eines der Führungsmittel (30, 31; 37, 39; 21; 23) drehbar ausgebildet ist.

40. Spinnereivorbereitungsmaschine nach einem der vorhergehenden Vorrichtungsansprüche, **dadurch gekennzeichnet, dass** zwischen dem Sensor (14) und einem Streckwerk (2) der Spinnereivorbereitungsmaschine (10) Umlenkelemente (42) zur Umlenkung von Faserbänder (1') des Faserverbandes (1) derart angeordnet sind, dass die Faserbänder (1') im wesentlichen die gleiche Wegstrecke zwischen dem Sensor (14) und dem Streckwerk (2) zurücklegen.

41. Spinnereivorbereitungsmaschine nach Anspruch 40, **dadurch gekennzeichnet, dass** mindestens zwei, in Faserverbandlängs- und -querrichtung gegeneinander versetzt angeordnete Umlenkelemente (42) zur Umlenkung mindestens eines Faserbandes (1') vorgesehen sind.

42. Spinnereivorbereitungsmaschine nach Anspruch 40 oder 41, **dadurch gekennzeichnet, dass** mindestens eines der Umlenkelemente (42) drehbar ausgebildet ist.

43. Spinnereivorbereitungsmaschine nach einem der vorhergehenden Vorrichtungsansprüche, **dadurch gekennzeichnet, dass** das Material zumindest des Probenvolumenabschnitts, der mit dem Faserverband (1) in Berührung kommt, im wesentlichen abriebfest ausgebildet ist.

44. Spinnereivorbereitungsmaschine nach einem der vorhergehenden Vorrichtungsansprüche, **gekennzeichnet durch** Mittel zum Erzeugen eines Saug- oder Blasluftstromes, der mit dem Probenvolumen des mindestens einen Resonators (14') zum Reinigen des Probenvolumens in Wirkverbindung bringbar ist.

45. Spinnereivorbereitungsmaschine nach einem der vorhergehenden Vorrichtungsansprüche, **dadurch gekennzeichnet, dass** zumindest das Probenvolumen des mindestens einen Resonators (14') verfahrbar ausgebildet ist.

46. Spinnereivorbereitungsmaschine nach einem der vorhergehenden Vorrichtungsansprüche, **dadurch gekennzeichnet, dass** sie als Karde mit einem regulierten Streckwerk oder als Kämmmaschine mit einem regulierten Streckwerk ausgebildet ist, wobei dem regulierten Streckwerk jeweils ein unreguliertes Streckwerk vorgeschaltet sein kann.

47. Spinnereivorbereitungsmaschine nach einem der vorhergehenden Vorrichtungsansprüche, **dadurch gekennzeichnet, dass** sie als Karde (50) oder Kämmmaschine ausgebildet ist, deren Auslauf ein reguliertes Streckwerk (2) in Form eines Moduls zustellbar ist.

48. Spinnereivorbereitungsmaschine nach einem der vorhergehenden Vorrichtungsansprüche, **dadurch gekennzeichnet, dass** sie als Karde (50) ausgebildet ist, an deren Auslauf anstelle eines mechanischen Wegmesssensors der mindestens eine Mikrowellensensor (14) angeordnet ist.

## Claims

1. Procedure for determining the band mass of a moved fibre structure in a spinning preparation machine (10) with several successive draw parts (21, 22, 23) for drawing the fibre structure (1), the procedure comprising the following steps:
- microwaves are coupled into at least one resonator (14') of a microwave sensor (14; 114),
- the fibre structure (1) is guided through or along the at least one resonator (14') in such a manner that the self resonance varies on account of the varying band mass and moisture of the fibre structure (1),
- the microwave frequency is adapted by processor-controlled coordination to the changed self resonance,
- the frequency-adapted microwaves coupled out of the at least one resonator (14') are detected as regards frequency detuning and damping,
- the band mass is calculated from the frequency detuning and the damping with the aid of a processor, taking into account the moisture of the fibre structure (1) by calculation.
**characterised in that** the scanning frequency the fibre structure guided through the resonator is scanned with is coordinated with the inlet speed of the fibre structure (1) entering the spinning preparation machine (10) or with the delivery speed of the fibre structure (1) leaving the spinning preparation machine (10) in such a way that a scanning length is kept which is independent of the sliver speed.

2. Procedure according to claim 1, **characterized in that** the scanning frequency is adapted to a fixed, preferably constant scanning length.

3. Procedure according to claim 1, **characterized in that** the scanning frequency is adapted to a fixed time span that is a function of the speed of the fibre structure.

4. Procedure according to one of the preceding claims, **characterized in that** the scanning detecting a certain fibre structure section per measurement is carried out along the fibre structure (1) in several overlapping measurements that are staggered relative to each other.

5. Procedure according to, **characterized in that** a spectrogram or a part of a spectrogram of the fibre structure (1') is prepared or supplemented using the measured valued obtained with the at least one microwave sensor (14, 114).

6. Procedure according to one of the preceding claims, **characterized in that** the resonance frequency corresponding to the particular band mass and the full width at half maximum of the signal at this resonance frequency are evaluated.

7. Procedure according to one of the preceding claims, **characterized in that** the microwave resonance measurements are performed at the inlet (12) and/or at the outlet (13) of a drafting system (2) of the spinning preparation machine (10).

8. Procedure according to one of the preceding claims, **characterized in that** the fluctuations of band mass at the inlet (12) and/or at the outlet (13) are monitored and, if necessary, the spinning preparation machine (10) is cut off and/or a warning signal emitted when threshold values of the band mass or of fluctuations of band mass are dropped below or exceeded.

9. Procedure according to one of the preceding claims, **characterized in that** the microwave sensor (14, 114) is designed to detect band breaks of the fibre structure (1) or of a sliver (1') of the fibre structure (1).

10. Procedure according to one of the preceding claims, **characterized in that** a regulating unit (16) of the spinning preparation machine (10) controls within the scope of an inlet regulation at least one of the draw parts (21, 22, 23) for stabilizing the fluctuations of sliver mass using the calculated values for the band mass.

11. Procedure according to one of the preceding claims, **characterized in that** a regulating unit (16) of the spinning preparation machine (10) regulates within the scope of an outlet regulation at least one of the draw parts (21, 22, 23) for stabilizing the fluctuations of sliver mass using the calculated values for the band mass.

12. Procedure according to claim 10 and claim 11, **characterized in that** the inlet regulation and outlet regulation form a meshed regulation.

13. Procedure according to one of the preceding claims, **characterized in that** the local fibre temperature of the fibre structure (1) is determined and that the local fibre temperature and/or the fibre moisture determined with the aid of the at least one microwave sensor is/are used in addition to the determined fibre density to control the spinning preparation machine.

14. Procedure according to claim 13, **characterized in that** a spectrogram of the fibre structure (1') is made at the inlet and/or the outlet of the spinning preparation machine.

15. Procedure according to one of the preceding claims, **characterized in that** the fibre structure (1) is guided substantially free of oscillations through the at least one resonator (14').

16. Procedure according to one of the preceding Procedure claims, **characterized in that** the fibre structure (1) is transported with lateral guidance through the at least one resonator (14').

17. Procedure according to one of the preceding claims, **characterized in that** the fibre structure (1) is guided through the at least one resonator (14') under tension and that the tension is realized by guide means (30, 31; 37, 39) in front of and behind the at least one resonator (14').

18. Procedure according to one of the preceding claims, **characterized in that** several fibre structures (1') are guided from the inlet (12) to the outlet (13) adjacent on each other and, viewed from above, running substantially in parallel through the spinning preparation machine.

19. Procedure according to one of the preceding claims, **characterized in that** the fibre structure (1) or individual groups (1 ") of slivers (1') forming the fibre structure (1) are guided through at least one funnel or by guide elements, e.g., guide sheets or guide rods before running through the at least one resonator (14').

20. Procedure according to one of the preceding claims, **characterized in that** the fibre structure (1) or individual groups (1") of slivers (1') forming the fibre structure (1) are guided via at least one guide means (37) with guide surfaces (38) rising to the sides before running through the at least one resonator (14') and that the fibre structure (1) or the slivers (1') of at least one group (1 ") are compressed.

21. Procedure according to one of the preceding claims, **characterized in that** the at least one resonator (14') is cleaned at time intervals with compressed air or a vacuum current.

22. Procedure according to one of the preceding claims, **characterized in that** at least the section of the at least one resonator (14') which section receives the fibre structure (1) is shifted out of its measuring position into a cleaning position during a fibre structure standstill for an automatic or manual cleaning.

23. A spinning preparation machine, especially a card (50), draw frame (10) or combing machine, especially for carrying out the procedure according to one of the preceding claims, with at least one sensor (14; 114) for measuring the band mass of continuously moved fibre structure (1), which at least one sensor (14; 114) comprising:
- at least one microwave generator (25) for producing microwaves of processor-controlled, variable frequencies,
- at least one microwave resonator (14') which the microwaves of variable frequency can be coupled into or out of,
- a specimen volume for receiving the fibre structure (1) as part of the microwave resonator (14'), and
- a processor to coordinate the microwave frequency that is coupled into with the self frequency of the microwave resonator (14') which has been changed by introducing the fibre structure (1) into the specimen volume
- an evaluation unit for evaluating the coupled out microwave resonance signals as regards the band mass of the fibre structure (1), with calculative consideration of the fibre structure moisture,
**characterized in that** the sensor is made ready to coordinate the scanning frequency, the fibre structure that is guided through the resonator is scanned with, with the inlet speed of the fibre structure (1) entering the spinning preparation machine (10) or with the delivery speed of the fibre structure (1) leaving the spinning preparation machine (10) in such a way that a scanning length is kept which is independent of said speed, while an external synchronisation entrance is provided receiving synchronisation signals that are dependent on the inlet speed or the delivery speed.

24. Spinning preparation machine according to claim 23, **characterized in that** the scanning frequency is adapted to a fixed, preferably constant scanning length.

25. Spinning preparation machine according to claim 23, **characterized in that** the scanning frequency is adapted to a fixed time span that is a function of the speed of the fibre structure.

26. Spinning preparation machine according to one of the preceding device claims, **characterized in that** it is designed such that the scanning detecting a certain fibre structure section per measurement is carried out along the fibre structure (1) in several overlapping measurements that are staggered relative to each other.

27. Spinning preparation machine according to one of the preceding device claims, **characterized in that** the at least one sensor (14) is arranged at the inlet of the spinning preparation machine.

28. Spinning preparation machine according to one of the preceding device claims, **characterized in that** the at least one sensor (14) is arranged at the outlet of the spinning preparation machine.

29. Spinning preparation machine according to one of the preceding device claims, **characterized in that** the at least one sensor (14) is connected to a regulating unit (16) that controls and/or regulates at least one draft part (21, 22, 23) of the spinning preparation machine using the measured values for the band mass of the fibre structure (1).

30. Spinning preparation machine according to one of the preceding device claims, **characterized in that** several slivers can be guided adjacent to each other and running in parallel through the at least one microwave sensor (14).

31. Spinning preparation machine according to one of the preceding device claims, **characterized in that** several slivers can be guided from the inlet to the outlet adjacent to each other and, viewed from the top, running substantially in parallel through the spinning preparation machine.

32. Spinning preparation machine according to one of the preceding device claims, **characterized in that** guide means (30, 31; 37, 39; 21; 23) are provided in front of and behind the sensor (14; 114) for guiding the fibre structure (1) with a tension.

33. The spinning preparation machine according to claim 32, **characterized in that** the guide means (30, 31; 37, 39; 21; 23) comprise rotating roller pairs (30, 31; 21; 23) between which the fibre structure (1) can be clamped.

34. Spinning preparation machine according to claim 32, **characterized in that** the space (x, y) between the roller pair (30, 31) arranged in front of and/or after the sensor (14) and the sensor (14) is small.

35. Spinning preparation machine according to one of the preceding device claims, **characterized in that** the guide means (30, 31; 37, 39; 21; 23) comprise at least one compression element designed as a funnel, guide sheets or guide rods in front of the at least one resonator (14') for bringing together the fibre structure (1) or individual groups (1 ") of slivers (1') of the fibre structure (1).

36. Spinning preparation machine according to one of the preceding device claims, **characterized in that** the guide means (30, 31; 37, 39; 21; 23) comprise at least one compression element (37) with guide surfaces (38) rising transversely to the longitudinal direction of the fibre structure for bringing together the fibre structure (1) or individual groups (1") of slivers (1') of the fibre structure (1).

37. Spinning preparation machine according to claim 36, **characterized in that** the at least one compression element (37) is designed substantially as a double cone with tips facing into one another and merging into one another.

38. Spinning preparation machine according to one of claims 32 to 37, **characterized in that** the guide means (30, 31; 37, 39; 21; 23) comprise a pressure rod (39) in front of the sensor (14).

39. Spinning preparation machine according to one of claims 32 to 38, **characterized in that** at least one of the guide means (30, 31; 37, 39; 21; 23) is designed to be rotatable.

40. Spinning preparation machine according to one of the preceding device claims, **characterized in that** deflection elements (42) for deflecting slivers (1') of the fibre structure (1) are arranged between the sensor (14) and a drafting system (2) of the spinning preparation machine (10) in such a manner that the slivers (1') traverse substantially the same path stretch between the sensor (14) and the drafting system (2).

41. Spinning preparation machine according to claim 40, **characterized in that** at least two deflection elements (42) for deflecting at least one sliver (1') are provided which elements are arranged staggered relative to each other in the longitudinal and the transverse direction of the fibre structures.

42. Spinning preparation machine according to claim 40 or 41, **characterized in that** at least one of the deflection elements (42) is designed to be rotatable.

43. Spinning preparation machine according to one of the preceding device claims, **characterized in that** the material at least of the specimen-volume section that makes contact with the fibre structure (1) is designed to be substantially wear-resistant.

44. Spinning preparation machine according to one of the preceding device claims, **characterized by** means for generating a current of suction or of air blast wind that can be brought into an operative connection with the specimen volume of the at least one resonator (14') for cleaning the specimen volume.

45. Spinning preparation machine according to one of the preceding device claims, **characterized in that** at least the specimen volume of the at least one resonator (14') is designed so that it can be moved.

46. Spinning preparation machine according to one of the preceding device claims, **characterized in that** it is designed as a card with a regulated drafting system or as a combing machine with a regulated drafting system and that an unregulated drafting system can be located in front of the regulated drafting system.

47. Spinning preparation machine according to one of the preceding device claims, **characterized in that** it is designed as a card (50) or combing machine at whose outlet a regulated drafting system (2) in the form of a module can be placed.

48. Spinning preparation machine according to one of the preceding device claims, **characterized in that** it is designed as a card (50) at whose outlet the at least one microwave sensor (14) is arranged instead of a mechanical path measuring sensor.

## Revendications

1. Procédé pour déterminer la masse de ruban d'un ensemble de fibres en mouvement à une machine de préparation de filage (10) comportant plusieurs organes d'étirage successifs (21, 22, 23) pour l'étirage de l'ensemble de fibres (1), sachant que le procédé comprend les étapes suivantes :
■ des microondes sont couplées dans au moins un résonateur (14') d'un capteur à microondes (14; 114),
■ l'ensemble de fibres (1) est déplacé à travers ou le long de l'au moins un résonateur (14') de telle manière que la résonance propre varie en fonction de la variation de la masse de ruban et de l'humidité de l'ensemble de fibres (1),
■ la fréquence des microondes est adaptée par syntonisation commandée par processeur à la nouvelle résonance propre,
■ les microondes découplées de l'au moins un résonateur (14') et adaptées en fréquence sont détectées en termes de distorsion de fréquences et d'affaiblissement.
■ un processeur est utilisé pour calculer la masse du ruban à partir de la distorsion de fréquence et de l'affaiblissement, en tenant compte, par le calcul, de l'humidité de l'ensemble de fibres (1),
**caractérisé en ce que** la fréquence d'échantillonnage avec laquelle est exploré l'ensemble de fibres déplacé à travers le résonateur est adaptée à la vitesse d'entrée de l'ensemble de fibres (1) pénétrant dans la machine de préparation du filage (10) ou à la vitesse de livraison de l'ensemble de fibres (1) sortant de la machine de préparation du filage (10), de telle manière que soit respectée une distance d'échantillonnage indépendante de la vitesse du ruban.

2. Procédé selon la revendication 1, **caractérisé en ce que** la fréquence d'échantillonnage est adaptée à une longueur d'échantillonnage déterminée, de préférence constante.

3. Procédé selon la revendication 1, **caractérisé en ce que** la fréquence d'échantillonnage est adaptée à un intervalle de temps déterminé, lequel dépend de la vitesse de l'ensemble de fibres.

4. Procédé selon l'une quelconque des revendications précédentes, **caractérisé en ce que** l'échantillonnage saisissant respectivement, à chaque mesure, un segment déterminé de l'ensemble de fibres, est effectué en plusieurs mesures décalées l'une par rapport à l'autre et se chevauchant le long de l'ensemble de fibres (1).

5. Procédé selon l'une quelconque des revendications précédentes, **caractérisé en ce qu'**un spectrogramme ou une partie d'un spectrogramme de l'ensemble de fibres (1') est établi ou complété sur la base des valeurs de mesure obtenues au moyen de l'au moins un capteur à microondes (14, 114).

6. Procédé selon l'une quelconque des revendications précédentes, **caractérisé en ce que** la fréquence de résonance correspondant respectivement à la masse de ruban ainsi que la largeur de valeur moyenne du signal à cette fréquence de résonance sont analysées.

7. Procédé selon l'une quelconque des revendications précédentes, **caractérisé en ce que** les mesures de résonance microondes sont effectuées à l'entrée (12) et/ou à la sortie (13) d'un banc d'étirage (2) de la machine de préparation du filage (10).

8. Procédé selon l'une quelconque des revendications précédentes, **caractérisé en ce que** les fluctuations de la masse de ruban à l'entrée (12) et/ou à la sortie (13) sont surveillées et, le cas échéant, la machine de préparation du filage (10) est arrêtée et/ou un signal d'avertissement est émis en cas d'écart en plus ou en moins par rapport à des valeurs seuils de masse de ruban ou de fluctuation de masse de ruban.

9. Procédé selon l'une quelconque des revendications précédentes, **caractérisé en ce que** le capteur à microondes (14, 114) se présente sous une forme permettant de détecter les ruptures de ruban de l'ensemble de fibres (1) ou d'un ruban de fibres (1') de l'ensemble de fibres (1).

10. Procédé selon l'une quelconque des revendications précédentes, **caractérisé en ce que** sur la base des valeurs calculées pour la masse de bande, une unité de régulation (16) de la machine de préparation du filage (10) régularise au moyen de réglage d'introduction au moins l'un des organes d'étirage (21, 22, 23) pour la compensation par régulation des fluctuations de la masse de bande.

11. Procédé selon l'une quelconque des revendications précédentes, **caractérisé en ce que** sur la base des valeurs calculées pour la masse de bande, une unité de régulation (16) de la machine de préparation du filage (10) régularise au moyen de réglage de sortie au moins l'un des organes d'étirage (21, 22, 23) pour la compensation par régulation des fluctuations de la masse de bande.

12. Procédé selon la revendication 10 et la revendication 11, **caractérisé en ce que** la régulation d'entrée et la régulation de sortie constituent une régulation maillée.

13. Procédé selon l'une quelconque des revendications précédentes, **caractérisé en ce que** la température locale des fibres de l'ensemble de fibres (1) est déterminées et que la température locale des fibres et/ou l'humidité des fibres déterminée au moyen de l'au moins un capteur à microondes sont utilisées en plus de la densité de fibres déterminée pour commander de la machine de préparation du filage.

14. Procédé selon la revendication 13, **caractérisé en ce qu'**un spectrogramme de l'ensemble de fibres (1') est relevé à l'entrée et/ou à la sortie de la machine de préparation du filage.

15. Procédé selon l'une quelconque des revendications précédentes, **caractérisé en ce que** l'ensemble de fibres (1) est conduit de manière essentiellement sans vibration à travers l'au moins un résonateur (14').

16. Procédé selon l'une quelconque des revendications précédentes, **caractérisé en ce que** l'ensemble de fibres (1) est transporté à travers l'au moins un résonateur (14') avec guidage latéral.

17. Procédé selon l'une quelconque des revendications précédentes, **caractérisé en ce que** l'ensemble de fibres (1) est conduit avec une tension à travers l'au moins un résonateur (14'), sachant que la tension est réalisée par des moyens de guidage (30, 31; 37, 39) en amont et en aval de l'au moins un résonateur (14').

18. Procédé selon l'une quelconque des revendications précédentes, **caractérisé en ce que** plusieurs rubans de fibres (1') sont conduits de l'entrée (12) à la sortie (13) l'un à côté de l'autre et, en vue de dessus, en translation essentiellement parallèle, à travers la machine de préparation du filage.

19. Procédé selon l'une quelconque des revendications précédentes, **caractérisé en ce que** l'ensemble de fibres (1) ou des groupes individuels (1") de rubans de fibres (1') constituant l'ensemble de fibres (1) sont conduits à travers au moins un entonnoir ou à travers des éléments de guidage, par exemple des tôles de guidage ou des barres de guidage, avant de traverser l'au moins un résonateur (14').

20. Procédé selon l'une quelconque des revendications précédentes, **caractérisé en ce que** l'ensemble de fibres (1) ou des groupes individuels (1") de rubans de fibres (1') constituant l'ensemble de fibres (1) sont conduits via au moins un moyen de guidage (37) avec des surfaces de guidage (38) ascendantes vers les côtés, sachant que l'ensemble de fibres (1) ou les rubans de fibres (1') d'au moins un groupe (1 ") sont comprimés, avant de traverser l'au moins un résonateur (14').

21. Procédé selon l'une quelconque des revendications de procédé précédentes, **caractérisé en ce que** l'au moins un résonateur (14') est nettoyé à des intervalles de temps au moyen d'air comprimé ou d'un flux d'aspiration.

22. Procédé selon l'une quelconque des revendications de procédé précédentes, **caractérisé en ce qu'**au moins la partie de l'au moins un résonateur (14') accueillant l'ensemble de fibres (1) est déplacée pour un nettoyage automatique ou manuel de sa position de mesure à une position de nettoyage lors d'une immobilisation de l'ensemble de fibres.

23. Machine de préparation de filage, particulièrement une cardeuse (50), une étireuse (10) ou une peigneuse, particulièrement pour la mise en application du procédé selon l'une quelconque des revendications précédentes, avec au moins un capteur (14, 114) pour mesure la masse de ruban d'un ensemble de fibres (1) déplacé continuellement, sachant que l'au moins un capteur (14, 114) comporte :
■ au moins un générateur de microondes (25) pour générer des microondes à fréquence commandée par processeur et variable,
■ au moins un résonateur à hyperfréquences (14'), dans lequel peuvent être couplées et découplées les hyperfréquences à fréquence variable,
■ un compartiment d'échantillon pour accueillir l'ensemble de fibres (1) comme partie du résonateur à hyperfréquences (14'),
■ un processeur pour la syntonisation de la fréquence de microondes couplée avec la résonance propre du résonateur à hyperfréquences (14'), modifiée par l'introduction de l'ensemble de fibres (1) dans le compartiment d'échantillon,
■ une unité d'exploitation pour l'analyse des signaux de résonance microondes découplés en termes de masse de ruban de l'ensemble de fibres (1) en tenant compte, par le calcul, de l'humidité de l'ensemble de fibres,
**caractérisée en ce que** le capteur est réglé de manière à accorder la fréquence d'échantillonnage avec laquelle est exploré l'ensemble de fibres déplacé à travers le résonateur, sur la vitesse d'entrée de l'ensemble de fibres (1) pénétrant dans la machine de préparation du filage (10) ou sur la vitesse de livraison de l'ensemble de fibres (1) sortant de la machine de préparation du filage (10) de telle manière que soit respectée une distance d'échantillonnage indépendante de ladite vitesse, sachant qu'une entrée de synchronisation externe est prévue, laquelle reçoit des signaux de synchronisation dépendant de la vitesse d'entrée ou de la vitesse de livraison.

24. Machine de préparation de filage selon la revendication 23, **caractérisée en ce que** la fréquence d'échantillonnage est adaptée à une longueur d'échantillonnage déterminée, de préférence constante.

25. Machine de préparation de filage selon la revendication 23, **caractérisée en ce que** la fréquence d'échantillonnage est adaptée à un intervalle de temps déterminé, lequel dépend de la vitesse de l'ensemble de fibres.

26. Machine de préparation de filage selon l'une quelconque des revendications de dispositif précédentes, **caractérisée en ce qu'**elle est conçue de telle manière que l'échantillonnage saisissant respectivement, à chaque mesure, un segment déterminé de l'ensemble de fibres, est effectué en plusieurs mesures décalées l'une par rapport à l'autre et se chevauchant le long de l'ensemble de fibres (1).

27. Machine de préparation de filage selon l'une quelconque des revendications de dispositif précédentes, **caractérisée en ce que** l'au moins un capteur (14) est disposé à l'entrée de la machine de préparation de filage.

28. Machine de préparation de filage selon l'une quelconque des revendications de dispositif précédentes, **caractérisée en ce que** l'au moins un capteur (114) est disposé à la sortie de la machine de préparation de filage.

29. Machine de préparation de filage selon l'une quelconque des revendications de dispositif précédentes, **caractérisée en ce que** l'au moins un capteur (14) est connecté à une unité de régulation (16), laquelle commande et/ou régule au moins un organe d'étirage (21, 22, 23) de la machine de préparation du filage sur la base des valeurs de mesure de la masse de ruban de l'ensemble de fibres (1).

30. Machine de préparation de filage selon l'une quelconque des revendications de dispositif précédentes, **caractérisée en ce que** plusieurs rubans de fibres peuvent être conduits l'un à côté de l'autre et en translation parallèle à travers l'au moins un capteur à microondes (14).

31. Machine de préparation de filage selon l'une quelconque des revendications de dispositif précédentes, **caractérisée en ce que** plusieurs rubans de fibres peuvent être conduits de l'entrée à la sortie l'un à côté de l'autre et, en vue de dessus, en translation essentiellement parallèle, à travers la machine de préparation du filage.

32. Machine de préparation de filage selon l'une quelconque des revendications de dispositif précédentes, **caractérisée en ce que** des moyens de guidage (30, 31; 37, 39; 21; 23) sont prévus en amont ou en aval du capteur (14; 114) pour guider l'ensemble de fibres (1) avec une certaine tension.

33. Machine de préparation de filage selon la revendication 32, **caractérisée en ce que** les moyens de guidage (30, 31; 37, 39; 21; 23) comportent des paires de cylindres rotatifs (30, 31; 21; 23), entre lesquelles l'ensemble de fibres (1) peut être serré.

34. Machine de préparation de filage selon la revendication 32, **caractérisée en ce que** la distance (x, y) de la paire de cylindres (30, 31) installée en amont et/ou en aval du capteur (14) au capteur (14) est faible.

35. Machine de préparation de filage selon l'une quelconque des revendications de dispositif précédentes, **caractérisée en ce que** les moyens de guidage (30, 31; 37, 39; 21; 23) comportent au moins un élément de compactage se présentant sous la forme d'un entonnoir, de tôles de guidage ou de barres de guidage, en aval de l'au moins un résonateur (14'), pour agglomérer l'ensemble de fibres (1) ou des groupes individuels (1") de rubans de fibres (1') de l'ensemble de fibres (1).

36. Machine de préparation de filage selon l'une quelconque des revendications de dispositif précédentes, **caractérisée en ce que** les moyens de guidage (30, 31; 37, 39; 21; 23) comportent au moins un élément de compactage (37) avec des surfaces de guidage (38) ascendantes perpendiculairement au sens longitudinal de l'ensemble de fibres, pour agglomérer l'ensemble de fibres (1) ou des groupes individuels (1") de rubans de fibres (1') de l'ensemble de fibres (1).

37. Machine de préparation de filage selon la revendication 36, **caractérisée en ce qu'**un élément de compactage (37) au moins se présente essentiellement sous la forme d'un double cône avec des sommets orientés l'un vers l'autre et se confondant l'un à l'autre.

38. Machine de préparation de filage selon l'une quelconque des revendications 32 à 37, **caractérisée en ce que** les moyens de guidage (30, 31; 37 ,39; 21; 23) englobent une éprouvette de compression (39) en amont du capteur (14).

39. Machine de préparation de filage selon l'une quelconque des revendications 32 à 38, **caractérisée en ce que** l'un au moins des moyens de guidage (30, 31; 37, 39; 21; 23) se présente sous une forme rotative.

40. Machine de préparation de filage selon l'une quelconque des revendications de dispositif précédentes, **caractérisée en ce que** des éléments déflecteurs (42) pour dévier des rubans de fibres (1') de l'ensemble de fibres (1) sont disposés entre le capteur (14) et un banc d'étirage (2) de la machine de préparation de filage (10) de manière à ce que les rubans de fibres (1') parcourent essentiellement la même distance entre le capteur (14) et le banc d'étirage (2).

41. Machine de préparation de filage selon la revendication 40, **caractérisée en ce que** deux éléments déflecteurs (42) au moins disposés en décalage l'un par rapport à l'autre dans les sens longitudinal et transversal de l'ensemble de fibres sont prévus pour dévier au moins un ruban de fibres (1').

42. Machine de préparation de filage selon l'une quelconque des revendications 40 ou 41, **caractérisée en ce que** l'un au moins des éléments déflecteurs (42) se présente sous une forme rotative.

43. Machine de préparation de filage selon l'une quelconque des revendications de dispositif précédentes, **caractérisée en ce que** la matière au moins de la section du compartiment à échantillon entrant en contact avec l'ensemble de fibres (1) se présente sous une forme essentiellement résistante à l'abrasion.

44. Machine de préparation de filage selon l'une quelconque des revendications de dispositif précédentes, **caractérisée par** des moyens pour générer un flux d'aspiration ou d'air de soufflage, lequel peut être mis en liaison fonctionnelle avec le compartiment d'échantillon de l'au moins un résonateur (14') pour nettoyer ledit compartiment d'échantillon.

45. Machine de préparation de filage selon l'une quelconque des revendications de dispositif précédentes, **caractérisée en ce qu'**au moins le compartiment d'échantillon de l'au moins un résonateur (14') se présente sous une forme mobile.

46. Machine de préparation de filage selon l'une quelconque des revendications de dispositif précédentes, **caractérisée en ce qu'**elle se présente sous la forme d'une cardeuse avec un banc d'étirage régulé ou d'une peigneuse avec un banc d'étirage régulé, sachant qu'un banc d'étirage non régulé peut être installé respectivement en amont du banc d'étirage régulé.

47. Machine de préparation de filage selon l'une quelconque des revendications de dispositif précédentes, **caractérisée en ce qu'**elle se présente sous la forme d'une cardeuse (50) ou d'une peigneuse et qu'un banc d'étirage (2) régulé peut être mis en position à sa sortie sous la forme d'un module.

48. Machine de préparation de filage selon l'une quelconque des revendications de dispositif précédentes, **caractérisée en ce qu'**elle se présente sous la forme d'une cardeuse (50) et que l'au moins un capteur à microondes (14) est disposé à sa sortie au lieu d'un capteur de mesure de déplacement mécanique.
